(19) Europäisches Patentamt
European Patent Office
Office européen des brevets

(11) Veröffentlichungsnummer: **0 239 816 B1**

(12) # EUROPÄISCHE PATENTSCHRIFT

(45) Veröffentlichungstag der Patentschrift: **11.05.94**

(51) Int. Cl.5: **C07D 251/52**, C08K 5/37

(21) Anmeldenummer: **87103018.5**

(22) Anmeldetag: **04.03.87**

(54) **N,N'-substituierte Bis-(2,4-diamino-s-triazin-6-yl)-Tetrasulfide und ihre Disproportionierungsprodukte, Verfahren zu ihrer Herstellung und ihre Verwendung in vulkanisierbaren Kautschukmischungen.**

(30) Priorität: **01.04.86 DE 3610794**

(43) Veröffentlichungstag der Anmeldung:
**07.10.87 Patentblatt 87/41**

(45) Bekanntmachung des Hinweises auf die Patenterteilung:
**11.05.94 Patentblatt 94/19**

(84) Benannte Vertragsstaaten:
**AT BE CH DE ES FR GB IT LI LU NL SE**

(56) Entgegenhaltungen:
**EP-A- 0 038 459**
**EP-A- 0 178 444**
**US-A- 3 923 724**

**RUBBER CHEMICALS, J. Van Alphen, 1973, Seiten 1-46, D. Reidel Publishing Co., Dordrecht, NL; "Accelerators"**

(73) Patentinhaber: **Degussa Aktiengesellschaft**
**Weissfrauenstrasse 9**
**D-60311 Frankfurt(DE)**

(72) Erfinder: **Schwarze, Werner, Dr.**
**Lerchesbergring 99**
**D-6000 Frankturt 70(DE)**
Erfinder: **Wolff, Siegfried**
**Weiherstrasse 28**
**D-5303 Bornheim-Merten(DE)**
Erfinder: **Lambertz, Horst**
**Kreuzstrasse 37**
**D-5030 Hürth(DE)**

EP 0 239 816 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft N,N′-substituierte Bis-(2,4-diamino-s-triazin-6-yl)-Tetrasulfide, ein Verfahren zu ihrer Herstellung, Verfahren zur Herstellung ihrer Disproportionierungsprodukte, die Verwendung der Tetrasulfide sowie der Disproportionierungsprodukte als Vernetzer oder Vulkanisationsbeschleuniger in Kautschukmischungen und sie enthaltende vulkanisierbare Kautschukmischungen.

N,N′-substituierte Bis-(2,4-diamino-s-triazin-6-yl) -Disulfide sind bekannt; sie sind in der DE-A-1 669 954 beschrieben. Man stellt sie aus den entsprechenden N,N′-substituierten 2,4-Diamino-6-mercaptotriazinen durch Oxidation, zum Beispiel mit Jod, Natriumhypochlorit oder Wasserstoffperoxid her. Die bekannteste Verbindung in dieser Gruppe ist das Bis-(2-ethylamino-4-di-ethylamino-triazin-6-yl)-Disulfid. Disulfide dieser Gruppe können in Kautschukmischungen als Beschleuniger verwendet werden.

Die Zielsetzung der Erfindung besteht darin, Verbindungen zu entwickeln, die das Vulkanisationsverhalten von Gummimischungen verbessern und ihren Vulkanisaten bessere Eigenschaften verleihen, und Verfahren zur Herstellung dieser Verbindungen.

Gegenstand der Erfindung sind

Verbindungen der allgemeinen Formel

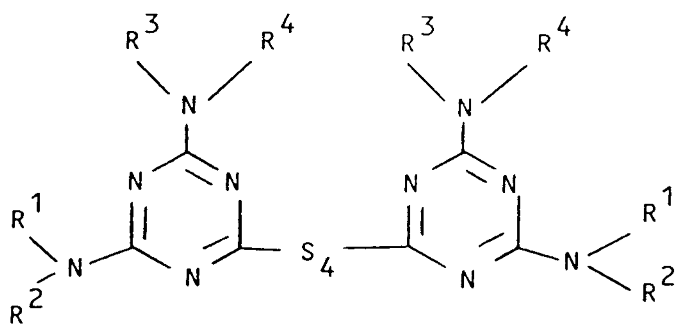

(I)

in welcher bedeuten:

$R^1$, $R^2$ = H, $R^2$ = Benzyl

$R^2$, $R^3$, $R^4$ = $C_1$-$C_8$-Alkyl, bevorzugt $C_1$-$C_4$-Alkyl, verzweigt oder unverzweigt, Allyl, $C_3$-$C_8$-Cycloalkyl, letzteres unsubstituiert oder mit 1-3 Methylgruppen substituiert, 2-Hydroxyethyl, 3-Hydroxypropyl, 2-Hydroxypropyl,

mit Ausnahme des in der EP-A-0 178 444 vorbeschriebenen aber nicht vorveröffentlichten Bis-(2-Ethylamino-4-diethylamino-s-triazin-6-yl)tetrasulfids Gegenstand der Erfindung ist ebenso ein Verfahren zur Herstellung dieser Verbindungen. Das Verfahren zur Herstellung der reinen Tetrasulfide mit einer linearen $S_4$-Kette zwischen den beiden substituierten Triazinresten ist dadurch gekennzeichnet, daß man eine wässrige, alkalische Lösung der entsprechenden N,N'-substituierten 2,4-Diamino-6-mercaptotriazinein einem Zweiphasensystem mit einer $S_2Cl_2$-Lösung in einem inerten organischen Lösungsmittel, in welchem das Reaktionsprodukt nicht oder sehr wenig löslich ist, bei Temperaturen zwischen -5 °C -< +20 °C, vorzugsweise von + 10 °C, umsetzt. Vorteilhaft stellt man eine alkalische wässrige Lösung des Mercaptotriazins her, die mindestens die zur Umsetzung notwendige stöchiometrische Menge an Alkalihydroxid, bevorzugt einen überschuß von 1 - 20 Mol%, bezogen auf das eingesetzte Mercaptotriazin, enthält.

Diese Lösung wird mit einem Lösungsmittel versetzt, in welchem das Endprodukt der Reaktion nicht oder wenig löslich ist, bevorzugt mit $C_5$-$C_{10}$-Alkanen, $C_5$-$C_8$ Cycloalkanen, gegebenenfalls substituiert mit 1 bis 3 Methylgruppen und deren Gemischen. Diese Mischung wird stark gerührt und abgekühlt, bevorzugt auf +10 °C. Nun läßt man in diese Mischung unter guter Kühlung eine Lösung von $S_2Cl_2$ in dem verwendeten Lösungsmittel zutropfen. $S_2Cl_2$ wird mindestens im Verhältnis 2 Mol Mercaptotriazin : 1 Mol $S_2Cl_2$ verwendet, doch kann dieses Verhältnis je nach Alkaliüberschuß auch 2: 1,1 - 1,2 betragen.

Unter den gegebenen Bedingungen wirkt $S_2Cl_2$ ausschließlichkondensierend.

Das entstandene Produkt wird mit Hilfe allgemein bekannter Maßnahmen abgetrennt und vorteilhaft bei Temperaturen bis zu +50 °C unter Vakuum $13{,}3 \cdot 10^2$ Pa (10 Torr) getrocknet.

Gegenstand der Anmeldung sind weiterhin Gemische, bestehend aus Verbindungen der allgemeinen Formel

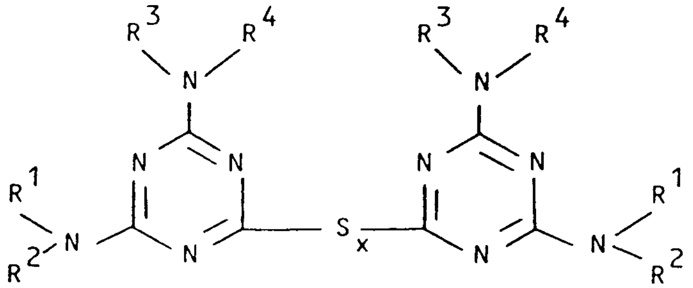

(II)

in der $R^1$, $R^2$, $R^3$ und $R^4$ dieselben Bedeutungen wie in Anspruch 1 haben und $S_x$ einer mittleren statistischen Kettenlänge mit x = 4 entspricht, mit Ausnahme des Bis-(2-Ethylamino-4-diethylamino-s-triazin-6-yl-)tetrasulfids.

Diese Gemische, im folgenden Text auch als Oligosulfide oder Disproportionate bezeichnet, da sie durch Disproportionierung von Verbindungen gemäß Formel I entstehen, können auf mehreren Wegen dargestellt werden.

Dabei sind die Reaktionsbedingungen so zu steuern, daß kein freier Schwefel entsteht.

Ein Verfahren ist dadurch gekennzeichnet, daß man die isolierte Verbindung gemäß Formel I über ihren Schmelzpunkt hinaus erhitzt, vorzugsweise um 20 - 50 °C.

In einem weiteren Verfahren löst man die Verbindungen gemäß Formel I in einem inerten organischen Lösungsmittel und führt die Disproportionierungsreaktion im Temperaturbereich zwischen 20 °C (Stehenlassen bei Zimmertemperatur) und Siedepunkt des eingesetzten Lösungsmittels durch.

Eine besonders elegante Methode besteht darin, daß man eine wässrige alkalische Lösung der entsprechenden N,N'-substituierten 2,4-Diamino-6-mercaptotriazine in einem Zweiphasensystem mit einer Lösung von $S_2Cl_2$ in einem inerten, das entstehende Tetrasulfid lösende organischen Lösungsmittel umsetzt. Das entstehende Tetrasulfid wird dann in der Folge sofort in das erfindungsgemäße Gemisch, bestehend aus Oligosulfiden, disproportioniert.

Als Lösungsmittel sind geeignet insbesondere chlorierte Kohlenwasserstoffe zum Beispiel $CH_2Cl_2$ und $CHCl_3$, aber auch Ether, Ester sowie aromatische Kohlenwasserstoffe und Ketone sind für die Disproportionierungsreaktion gemäß Anspruch 8 geeignet. Gemäß Anspruch 9 sind sie einsetzbar, wenn sie mit Wasser ein zweiphasiges Gemisch bilden. Die Reaktionsbedingungen zur Herstellung der Disproportionate sind ansonsten identisch mit denen zur Herstellung von Verbindungen gemäß Formel I.

Gegenstand der Erfindung sind ebenso die Verwendung der beanspruchten Verbindungen in vulkanisierbaren Kautschukmischungen und die die erfindungsgemäßen Verbindungen enthaltenden Kautschukmischungen selbst.

Die erfindungsgemäßen Tetrasulfide beziehungsweise deren Disproportionate zeigen sich bei ihrer Verwendung als Vernetzer beziehungsweise Vulkanisationsbeschleuniger den heute verwendeten Standardverbindungen als deutlich überlegen.

Der kautschukverarbeitenden Industrie steht eine umfangreiche Palette von Beschleunigern (J. van Alphen, Rubber Chemicals (1977, S. 1-46)), vorzugsweise für die Schwefelvulkanisation, zur Verfügung, zum Beispiel Benzthiazolylsulfenamide, Benzthiazolyldisulfid und 2-Mercaptobenzthiazol bzw. deren Zinksalze. Daneben gibt es eine Reihe spezieller Verbindungen wie Thiuramdisulfide und Peroxide, die auch ohne weitere Zusatzstoffe von Schwefel als Vernetzer wirken, oft aber auch in Kombination mit Schwefel als Beschleuniger eingesetzt werden. Die Art des Einsatzes hängt vom jeweils zu erzielenden Effekt ab.

Die auch mengenmäßig größte Bedeutung in der praktischen Anwendung, insbesondere bei den der beschleunigten Schwefelvulkanisation zugänglichen Elastomeren, kommt heute den Benzthiazolylsulfenamiden zu.

Indessen haben sich durch neue Produktionsverfahren sowie neue Artikel und durch den Zwang zu ständiger Rationalisierung in den letzten Jahren die Einsatzanforderungen für Beschleuniger gegenüber früher in einem Maße geändert, daß es heute bereits Schwierigkeiten bereiten kann, den gestellten Qualitätsanforderungen an den Vulkanisationsprozeß sowie die Eigenschaften der Vulkanisate mit den heute für die beschleunigte Schwefelvulkanisation zur Verfügung stehenden Beschleunigern beziehungsweise Vernetzern nachzukommen.

Ein weiterer heute nicht mehr zu vernachlässigender Nachteil mancher konventioneller Beschleuniger (z.B. mancher Sulfenamide, Thiurame) liegt darin, daß während des Vulkanisationsprozesses Amine freigesetzt werden können, die - soweit sie nitrosierbar sind - zur Bildung von Nitrosaminen im Vulkanisat führen, die - soweit sie toxisch sind - auf Dauer eine Beschränkung der Einsatzmöglichkeiten dieser Beschleuniger erwarten lassen.

Ein ganz wesentlicher Nachteil der Benzthiazolylbeschleuniger, insbesondere der Benzthiazolylsulfenamide ist ihre mit steigender Vulkanisationstemperatur immer stärker ausgeprägte Neigung zur Reversion beim zwangsläufig oft notwendigen Überheizen der Vulkanisate, besonders bei Verwendung ohnehin reversionsanfälliger Kautschukarten wie Naturkautschuk und Polyisopren sowie deren Verschnitte mit Synthesekautschuken. Ähnliches gilt aber auch für Synthesekautschuke aller Art, sofern der Reversionsprozeß nicht durch thermische Vernetzung überdeckt wird. Besonders mit steigender Vulkanisationstemperatur nimmt die Reversionsgeschwindigkeit so stark zu, daß es erstens schon zu einer merklichen Absenkung der Vernetzungsdichte auch bei optimaler Vulkanisation an sicht kommt, daß zweitens das Vulkanisationsoptimum statt eines Plateaus die Form eines Peaks annimmt, was die reproduzierbare Einhaltung optimaler Vulkanisateigenschaften ungemein erschwert und daß drittens bei der in vielen Fällen notwendigen Übervulkanisation ein unvermeidbarer Abfall der Vernetzungsdichte eintritt, was insbesondere bei dickwandigen Gummiartikeln zur Aufhebung der Gleichmäßigkeit der Vernetzung im Vulkanisat führt. Im gleichen Maße, wie die Reversion zunimmt, kommt es zu einem Leistungsabfall der Vulkanisate, der sich z.B. in vermindertem 300%-Modul und Abriebwiderstand usw. äußert.

In gewissem Maße führt die Reduktion des Schwefel- und die Erhöhung des Beschleunigeranteils, d.h. der Einsatz sogenannter Semi-EV-Systeme (L. Bateman, 1963 "The Chemistry and Physics of rubber-like substances", S. 522 ff), zu einer Abmilderung der geschilderten Reversionseffekte. Diese Abmilderung der Reversion wird aber über eine Änderung der Vernetzungsstruktur (Verhältnis von $-S_x-$,-S-S-, -S-Bindungen) zugunsten monosulfidischer Vernetzungsstellen erzielt, die die Vulkanisateigenschaften nachteilig beeinflussen kann. Indessen wird diese Maßnahme immer unwirksamer, je höher die Vulkanisationstemperatur gesetzt wird.

Diese Nachteile der Benzthiazolbeschleuniger schränken ihre Verwendbarkeit mit steigender Vulkanisationstemperatur ein und setzen den Bestrebungen der Kautschukindustrie zur Produktivitätssteigerung durch Verwendung höherer Vulkanisationstemperaturen bestimmte Grenzen.

Überraschenderweise erweisen sich die erfindungsgemäßen N,N′-substituierten Bis-(2,4-diamino-s-triazin-6-yl)-Tetrasulfide sowie deren Disproportionierungsprodukte sowohl hinsichtlich ihrer Verwendung als Beschleuniger bei der Schwefelvulkanisation als auch bei ihrer Verwendung als Vernetzer, d.h. ohne den zusätzlichen Einsatz von Schwefel, als Verbindungen, die den damit hergestellten Kautschukmischungen auch bei hohen Vulkanisationstemperaturen außerordentlich hohe Reversionsstabilität - auch bei sehr starker Überheizung - verleihen, dadurch daß der bei ihrer Verwendung sonst übliche Reversionsprozeß entweder überhaupt nicht auftritt oder wenn, dann nur in geringem Maße. Die überraschende Tatsache, daß die durch die erfindungsgemäß beanspruchten N,N′-substituierten Bis-(2,4-diamino-s-triazin-6-yl)-Tetrasulfide bzw. deren Disproportionate entstehenden Vernetzungsstellen sich als außerordentlich reversions- und thermostabil erweisen, führt bei ihrem Einsatz in Kautschukmischungen auch bei hohen Vulkanisationstemperaturen dazu, daß nach Abschluß der Vernetzungsreaktion einerseits ein hohes Leistungsniveau der Vulkanisate erreicht wird und andererseits dazu, daß auch bei starker Überheizung das hohe Leistungsniveau lange erhalten bleibt.

Diese beiden Effekte zusammen genommen ermöglichen in der kautschukverarbeitenden Industrie durch Anhebung der Vulkanisationstemperatur Produktivitätssteigerungen ohne Vulkanisat-Leistungsverlust.

Eine auch nur annähernd gleiche Reversionsbeständigkeit läßt sich mit handelsüblichen Polysulfiden wie Robac® P 25 (Robinson, Dipentamethylen-Thiuramtetrasulfid) und Tetron®A (Du Pont, Dipentamethylen-Thiuramhexasulfid) oder mit Dibenzthiazolyl-Tetrasulfid nicht erzielen.

Die Verwendung der erfindungsgemäß beanspruchten N,N′-substituierten Bis- (2,4-diamino-s-triazin-6-yl)-Tetrasulfide sowie deren Disproportionierungsprodukte umfaßt die nach dem Stand der Technik bekannten Kautschukmischungen auf der Basis von Naturkautschuk (NR), Isoprenkautschuk (IR), Butadienkautschuk (BR) Styrol-Butadienkautschuk (SBR), Isobutylen-Isoprenkautschuk (IIR), Ethylen-Propylen-Terpolymer (EPDM), Nitrilkautschuk (NBR), halogenhaltige Kautschuke und auch epoxidierte Naturkautschuke (ENR) sowie deren Verschnitte. Besondere Bedeutung hat die Verwendung der erfindungsgemäß beanspruchten N,N′-substituierten Bis-(2,4-diamino-s-triazin-6-yl)-Tetrasulfide und deren Disproportionierungsprodukte im Falle der reversionsanfälligen Kautschüktypen wie zum Beispiel Naturkautschuk, Isopren- und Butadien-kautschuke, sowie deren Verschnitte untereinander oder mit anderen Kautschuken.

Die erfindungsgemäß beanspruchten N,N′-substituierten Bis-(2,4-diamino-s-triazin-6-yl)-Tetrasulfide und deren Disproportionierungsprodukte werden als Vernetzer in Kautschukmischungen in einer Menge von 0,2

- 15 Tln., bevorzugt 0,3 - 8 Gewichtsteilen, auf 100 Teile Kautschuk, eingesetzt.

Bei der beschleunigten Schwefelvulkanisation setzt man die erfindungsgemäß beanspruchten N,N′-substituierten Bis-(2,4-diamino-s-triazin-6-yl)-Tetrasulfide oder deren Disproportionierungsprodukte als Beschleuniger in Mengen von 0.01 - 10 Tln., vorzugsweise 0.1 - 5 Tln. bezogen auf 100 Tle. Kautschuk, ein bei Schwefeldosierungen von 0.1 - 10 Tln. Bevorzugt wird ein molares Verhältnis der erfindungsgemäßen Beschleuniger zu Schwefel ($S_8$) von 1 : 0,5 - 1,5. Zur Erzielung einer gewissen Variationsbreite der Vulkanisationskinetik kann es sich dabei als zweckmäßig erweisen, 2 oder mehrere N,N′-substituierte Bis-(2,4-diamino-s-triazin-6-yl)-Tetrasulfide oder deren Disproportionate im Gemisch einzusetzen, wobei zur Einhaltung der o.g. Anwendungsmengen, insbesondere des bevorzugten Beschleuniger/Schwefelverhältnisses, die Substitution auf molarer Basis vorzunehmen ist. Gleiches gilt bei Verwendung der N,N′-substituierten Bis-(2,4-diamino-s-triazin-6-yl)-Tetrasulfide oder deren Disproportionaten als Vernetzer ohne Schwefel.

Ebenfalls aus kinetischen Gründen kann es sich als zweckmäßig erweisen, die N,N′-substituierten Bis-(2,4-diamino-s-triazin-6-yl)-Tetrasulfide bzw. deren Disproportionierungsprodukte im Gemisch mit konventionellen Beschleunigern wie z.B. Sulfenamiden und Thiuramen zu verwenden. Diese Maßnahmen gehen gelegentlich zu Lasten der Reversionsbeständigkeit im Vergleich zu den N,N′-substituierten Bis-(2,4-diamino-s-triazin-6-yl)-Tetrasulfid Vulkanisaten, indessen wirkt sie sich hinsichtlich des Reversionsverhaltens umgekehrt positiv aus, wenn man konventionelle Beschleuniger partiell durch N,N′-substituierte Bis-(2,4-diamino-s-triazin-6-yl)-Tetrasulfide oder deren Disproportionate ersetzt.

Eine weitere wesentliche Beeinflussung der Inkubationszeit bei Verwendung der erfindungsgemäßen Verbindungen in Kautschukmischungen kann durch Kombination mit handelsüblichen Vulkanisationsverzögerern, wie Santoguard® PVI (N-(Cyclohexylthio)-Phthalimid), Vulkalent® E (N-Phenyl-N-(trichlormethylsulfenyl)-benzolsulfonamid), erreicht werden. Als effektivster Verzögerer hat sich das Vulkalent® E der Fa. Bayer AG erwiesen. Mit steigendem Vulkalent® E-Zusatz erfolgt ein linearer Anstieg der Inkubationszeit N,N′-substituierter Bis-(2,4-diamino-s-triazin-6-yl)-Tetrasulfide bzw. deren Disproportionate enthaltende Mischungen.

Bei Verwendung N,N′-substituierter Bis-(2,4-diamino-s-triazin-6-yl)-Tetrasulfide bzw. ihrer Disproportionierungsprodukte als Vernetzer hat sich die Einhaltung des molaren Verhältnisses von N,N′-substituierten Bis-(2,4-diamino-s-triazin-6-yl)-Tetrasulfiden bzw. deren Disproportionaten zu Vulkalent E von 1 : 0.5 - 1.5 vorzugsweise 0.8 - 1.2 Tln. auf 100 Tle. Kautschuk als zweckmäßig erwiesen.

Von größerer Bedeutung ist der Einsatz von Verzögerern, insbesondere Vulkalent E, bei der beschleunigten Schwefelvulkanisation unter Verwendung von N,N′-substituierten Bis-(2,4-diamino-s-triazin-6-yl)-Tetrasulfiden und ihrer Disproportionate sowie Gemischen derselben. Dabei beobachtet man gelegentlich neben der beabsichtigten Verlängerung der Inkubationszeit der Vernetzungsreaktion eine geringe Beeinträchtigung der Vernetzungsgeschwindigkeit, die aber durch Temperaturerhöhung aufgefangen werden kann und einen geringfügigen Abfall der Reversionsbeständigkeit. Es hat sich in diesem Fall ebenfalls als zweckmäßig herausgestellt, das molare Verhältnis von N,N′-substituierten Bis-(2,4-diamino-s-triazin-6-yl)-Tetrasulfiden bzw. deren Disproportionate zu Vulkalent E auf 1 : 0.5 - 1.5 einzustellen, vorzugsweise 1 : 0.8 - 1.2, wobei der Schwefelgehalt je nach Mischungstyp zwischen 0.1 - 10 Tln., vorzugsweise 0.5 - 8 Tln. bezogen auf 100 Tle. Kautschuk, zu halten ist.

Mit diesen Dosierungsrichtlinien lassen sich viele der gestellten Vulkanisationsprobleme lösen, ohne daß dabei ein dem Wesen der Erfindung widersprechender wesentlicher Vulkanisateigenschaftsverlust eintritt.

Mischungen, die nur Kieselsäure enthalten oder Ruß/Kieselsäure-Verschnitte mit höheren Anteilen an Kieselsäuren als 15 Tln./100 Tln. Kautschuk, sind, wenn überhaupt, mit konventionellen Schwefelvulkanisationssystemen nur schwer zu vernetzen. Einerseits unterliegen sie besonders der Reversion, andererseits verhindern sie die Einstellung der Vernetzungsdichte, die durch die Anforderungen an Fertigartikel vorgegeben ist. Dies ist einer der Gründe, weshalb Kieselsäuren bevorzugt in Besohlungsmaterial eingesetzt werden und bis heute relativ selten in dynamisch beanspruchten Artikeln.

Überraschenderweise gelingt es mit Bis-(2,4-diamino-s-triazin-6-yl)-tetrasulfiden und ihren Disproportionaten bei Mischungen, die Schwarz/Weiß-Verschnitte mit Kieselsäureanteilen von mehr als 15 Tln./100 Teile Kautschuk enthalten und auch mit Kieselsäure-gefüllten Mischungen sowohl bei Verwendung als Beschleuniger wie auch als Vernetzer (ohne Schwefel) die Reversion praktisch zu eliminieren und gleichzeitig die Vernetzungsdichte zu erhöhen, was in einem für Kieselsäure-Mischungen hohen Spannungsniveau zum Ausdruck kommt.

Eine weitere Anwendung der N,N′-substituierten Bis-(2,4-diamino-s-triazin-6-yl)-Tetrasulfide und ihrer Disproportionierungsprodukte besteht im kombinierten Einsatz mit oligosulfidischen Organo-Silanen, zum Beispiel

$[(RO)_3 - Si - (CH_2)_n-]_2 -S_x$ oder $(RO)_3-Si(CH_2)_n-SH$

mit
x = 2 - 6, R = $C_1$-$C_6$-Alkyl, Cyclohexyl
n = 2 oder 3
beziehungsweise

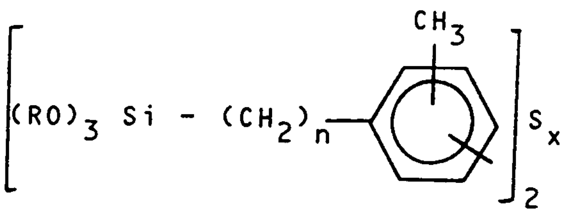

mit
x = 2 - 6, bevorzugt 3,
vorzugsweise Bis-(3-triäthoxisilylpropyl)-Tetrasulfid (Si 69, Degussa AG). N,N'-substituierte Bis-(2,4-diamino-s-triazin-6-yl)-Tetrasulfide und deren Disproportionate lassen sich sowohl bei der schwefelfreien Organosilan-Vernetzung anstelle der in DE-A-25 36 674 beschriebenen Beschleuniger vorteilhaft verwenden als auch bei der in DE-A-22 55 577 beschriebenen Schwefelvulkanisation mit Organosilanen und ebenso bei Herstellung reversionsstabiler Organosilan-haltiger Kautschukmischungen durch Aufbau von equilibrium cure systems gemäß DE-A-28 48 559.

Dies gilt sowohl für rußgefüllte Mischungen als auch für Mischungen, die Ruß/Kieselsäure-Verschnitte enthalten, als auch für Mischungen, die nur unter Verwendung von Kieselsäuren aufgebaut sind. Auch die Zugabe mineralischer Füllstoffe wirkt sich in keiner der genannten Mischungen nachteilig aus. Dabei werden in Gegenwart von Kieselsäurefüllstoffen oder von Verschnitten von Ruß mit Kieselsäuren Kautschuk/Füllstoff-Bindungen gebildet (Kautschuk + Gummi, Kunststoffe, Heft 8, 1977, S. 516-523, Heft 10, 1979, S. 760-765 und Heft 4, 1981), S. 280-284) oder in Gegenwart von Rußen Kautschuk/Kautschuk-Bindungen.

Der Aufbau von Kautschuk/Kautschuk-Vernetzungsstellen in Gegenwart von Ruß oder der von Kautschuk/Füllstoff-Vernetzungsstellen in Gegenwart von Kieselsäure sowie beider Bindungsarten im Falle von Ruß/Kieselsäure-Verschnitten aller Verhältnisse gelingt auch, wenn N,N'-substituierte Bis-(2,4-Diamino-s-triazin-6-yl)-tetrasulfide oder deren Disproportionate alleine oder im Gemisch mehrerer oder im Gemisch mit konventionellen Beschleunigern zusammen mit konventionellen Schwefelspendern wie zum Beispiel Sulfasan$^{(R)}$ R (Morpholindisulfid) eingesetzt werden.

Die Verwendung der erfindungsgemäßen Verbindung gemäß Ansprüchen 1 bis 11 erfolgt in Kautschukmischungen, die weitere übliche Komponenten enthalten können wie zum Beispiel:

- übliche Verstärkungssysteme, d.h. Furnace-Ruß, Channel-Ruße, Flammruße, Thermalruße, Acetylenruße, Lichtbogenruße, CK-Ruße usw. sowie synthetische Füllstoffe wie Kieselsäuren, Silikate, Aluminiumoxidhydrate, Calciumcarbonate und natürliche Füllstoffe wie Clays, Kieselkreiden, Kreiden, Talke usw. sowie silanmodifizierte Füllstoffe und deren Verschnitte in Mengen von 5 - 300 Tln. je 100 Tle. Kautschuk, bevorzugt sind insbesondere Kieselsäuren und Silikate,
- ZnO und Stearinsäure als Promotoren der Vulkanisation in Mengen von 0.5 - 10 Tln. Kautschuk,
- üblicherweise verwendete Alterungs-, Ozon-, Ermüdungsschutzmittel wie zum Beispiel IPPD, TMQ sowie auch Wachse als Lichtschutzmittel und deren Verschnitte.
- beliebige Weichmacher wie zum Beispiel aromatische, naphthenische, paraffinische, synthetische Weichmacher und deren Verschnitte,
- ggfs. weitere Silane wie -Chlorpropyltrialkoxisilane, Vinaltrialkoxysulane, und Aminoalkyltrialkoxisilane, sowie deren Verschnitte in einer Menge von 0.1 - 15, bevorzugt 1 - 10 Tle. je 100 Tle. silanolgruppentragender Füllstoffe wie Kieselsäuren, Silikate, Clays usw.
- ggfs. Farbstoffe und Verarbeitungshilfsmittel in der üblichen Dosierung.

Der Anwendungsbereich der N,N'-substituierten Bis-(2,4-diamino-s-triazin-6-yl)-Tetrasulfide erstreckt sich auf Kautschukmischungen gemäß den Ansprüchen 13 bis 16 wie sie üblicherweise im Reifenbau verwendet werden und auf technische Artikel, wie zum Beispiel Mischungen für Fördergurte, Keilriemen, Formartikel, Schläuche mit und ohne Einlagen, Walzengummierungen, Auskleidungen, Spritzprofile, Freihandartikel, Folien, Schuhsohlen und Oberteile, Kabel, Vollgummireifen und deren Vulkanisate.

Als Beispiel erfindungsgemäßer Verbindungen seien die folgenden Produkte genannt:

A Bis-(2-ethylamino-4-di-isopropylamino-s-triazin-6-yl)-tetrasulfid
B Bis-(2-n-butylamino-4-diethylamino-s-triazin-6-yl)-tetrasulfid
C Bis-(2-isopropylamino-4-di-isopropylamino-s-triazin-6-yl)-tetrasulfid
D Bis-(2-ethylamino-4-di-isobutylamino-s-triazin-6-yl)-tetrasulfid
E Bis-(2-ethylamino-4-di-n-propylamino-s-triazin-6-yl)-tetrasulfid
F Bis-(2-n-propylamino-4-diethylamino-s-triazin-6-yl)-tetrasulfid
G Bis-(2-n-propylamino-4-di-n-propylamino-s-triazin-6-yl-)-tetrasulfid
H Bis-(2-n-butylamino-4-di-n-propylamino-s-triazin-6-yl)-tetrasulfid
I Bis-(2-ethylamino-4-di-n-butylamino-s-triazin-6-yl)-tetrasulfid
K Bis-(2-isopropylamino-4-di-isopropylamino-s-triazin-6-yl)-oligosulfidgemisch
L Bis-(2-cyclohexylamino-4-diethylamino-s-triazin-6-yl)-oligosulfidgemisch
M Bis-(2-ethylamino-4-diethylamino-s-triazin-6-yl)-oligosulfidgemisch
O Bis-(2-amino-4-diethylamino-s-triazin-6-yl)-oligosulfidgemisch

Referenz - Beispiel 1:

Es handelt sich hierbei um das in der EP-A-0 178 444 vorbeschriebene Beispiel 1.

Man löst 454 g 2-Ethylamino-4-diethylamino-6-mercaptotriazin in Natronlauge, die man aus 84 g NaOH + 1,5 Liter $H_2O$ hergestellt hat.

Die Lösung gibt man in einen 4 Liter 3 Tubenkolben, dann gibt man 1,5 Liter Leichtbenzin (Kp 80 - 110 °C) hinzu und kühlt die Mischung unter starkem Rühren auf 0 °C ab.

Nun läßt man innerhalb von 20 min. eine Lösung von 137 g $S_2Cl_2$ in100 ml Benzin ein laufen, wobei man darauf achtet, daß die Temperatur +5 °C nicht überschreitet.

Das Tetrasulfid fällt sofort aus. Am Ende der Reaktion wird 5 min. nachgerührt, anschließend abgenutscht und gewaschen.

Das schneeweiße feine Pulver wird im Vakuum $15{,}96 \cdot 10^2$ Pa (12 Torr) bei 40 - 45 °C getrocknet.
Menge: 499,5 g, entsprechend 97,1 % der Theorie,
F. 149 - 150 °C.

Analyse:
Bis-(2-ethylamino-4-diethylamino-s-triazin-6-yl)-Tetrasulfid,
Molgew. 516, $C_{18}H_{32}N_{10}S_4$

| | | | | |
|---|---|---|---|---|
| ber. | C 41,9 | H 6,2 | N 27,1 | S 24,8 |
| gef. | 41,8 | 6,5 | 26,8 | 24,8 |

TLC- und HPLC-Analyse zeigen, daß das Produkt 97,1 % lineares Tetrasulfid enthält.

Beispiel 2:

Man löst 56,6 g 2-Ethylamino-4-di-n-butylamino-6-mercaptotriazin in einer Lösung von 8,8 NaOH in 250 ml Wasser. Dazu gibt man 250 ml Benzin. Die Mischung wird nach gutem Rühren auf +5 °C abgekühlt. Nun läßt man eine Lösung von 13,5 g $S_2Cl_2$ in 30 ml Benzin zulaufen. Es bildet sich sofort ein weißer Niederschlag. Am Ende der Reaktion wird nach Beispiel 1 aufgearbeitet.
Menge: 56 g, entsprechend 89,2 % der Theorie.

| Analyse: $C_{26}H_{48}N_{10}S_4$ (Molgew. 628) | | | | | | | | |
|---|---|---|---|---|---|---|---|---|
| ber.<br>gef. | C | 49,68<br>49,59 | H | 7,64<br>7,59 | N | 22,29<br>22,18 | S | 20,38<br>20,40 |

HPLC-Analyse: Reinheitsgrad >96 %.

Beispiel 3:

107,6 g 2-i-Propylamino-4-diisopropylamino-6-mercaptotriazin löst man in Natronlauge, die man aus 17,6 g NaOH in 600 ml $H_2O$ herstellt. Dazu gibt man 600 ml Methylenchlorid.

Bei 0-5°C läßt man eine Lösung von 27 g $S_2Cl_2$ in 50 ml $CH_2Cl_2$ zulaufen. Am Ende der Reaktion trennt man in einem Scheidetrichter die organische Phase ab, trocknet und dampft im Vakuum ein. Man erhält ein amorphes Pulver; Erweichungspunkt: 90°C.
Ausbeute: 112,5 g, entsprechend 94 % der Theorie.

| Analyse: $C_{24}H_{44}N_{10}S_4$ (Molgew. 600) | | | | | | | | |
|---|---|---|---|---|---|---|---|---|
| ber.<br>gef. | C | 48<br>48,2 | H | 7,33<br>7,36 | N | 23,3<br>23,01 | S | 21,3<br>20,95 |

Beispiel 4:

Man löst 45,4 g 2-Ethylamino-4-diethylamino-6-mercaptotriazin in Natronlauge, hergestellt aus 8,8 g NaOH und 200 ml Wasser. Dazu gibt man 200 ml Methylenchlorid. Die Mischung wird turbiniert und auf 0°C abgekühlt. Man löst nun 14 g $S_2Cl_2$ in 50 ml $CH_2Cl_2$ und läßt diese Lösung in die Mercaptidlösung einlaufen.

Das Reaktionsprodukt löst sich in $CH_2Cl_2$. Am Ende der Reaktion trennt man die Phasen und arbeitet die $CH_2Cl_2$-Lösung auf. Man erhält so ein amorphes Pulver mit einem Erweichungspunkt von ca. 110°C.
Menge: 46,7 g, entsprechend 90,5 % der Theorie.

| Analyse: $C_{18}H_{32}N_{10}S_4$ (Molgew. 516) | | | | |
|---|---|---|---|---|
| ber.<br>gef. | N | 27,1<br>26,8 | S | 24,8<br>24,4 |

Laut TLC-Analyse enthält man ein Gemisch aus 4 Oligosulfide,aber keinen freien Schwefel

Beispiel 5:

Man gibt 50 g Bis-(2-ethylamino-4-diethylamino-s-triazin-6-yl)-Tetrasulfid mit einem Reinheitsgrad von 97,1 % in einen Rundkolben und erwärmt im Ölbad 1 Stunde auf 160°C. Kalt erstarrt die Schmelze amorph. Nach TLC-Analyse enthält das Produkt neben ca. 50 % Ausgangsprodukt 3 weitere Oligosulfide.

Beispiel 6:

70,25 g 2-Cyclohexylamino-4-diethylamino-6-mercaptotriazin löst man in 11 g NaOH und 250 ml Wasser. Dazu gibt man 250 ml Chloroform. Unter starkem Rühren läßt man eine Lösung von 16,8 g $S_2Cl_2$ in 30 ml $CHCl_3$ zulaufen. Am Ende der Reaktion trennt man die Phase und arbeitet die Chloroformschicht auf. Man erhält 71,9 g eines weißen amorphen Pulvers, entsprechend 92 % der Theorie.

| Analyse: $C_{26}H_{44}N_{10}S_4$ (Molgew. 624) | | | | | | | | |
|---|---|---|---|---|---|---|---|---|
| ber.<br>gef. | C | 50<br>49,1 | H | 7,05<br>6,90 | N | 22,4<br>21,8 | S | 20,51<br>20 |

Laut TLC-Analyse setzt sich das Produkt aus ca. 30% linearem $S_4$-Produkt und 70 % Oligosulfiden zusammen, enthält aber keinen freien Schwefel.

Prüfungsnormen

Die physikalischen Prüfungen wurden bei Raumtemperatur nach folgenden Normvorschriften ausgeführt:

| | | gemessen in |
|---|---|---|
| Zugfestigkeit, Bruchdehnung und Spannungswert an 6 mm starken Ringen | DIN 53 504 | MPa |
| Shore-A-Härte | DIN 53 505 | - |
| Firestone-Ball Rebound | AD 20245 | % |
| Reversion | DE-PS 2 848 559 | |
| Inkubationszeit $t_i$ | DIN 53529 | (min) |
| Scorchzeit | ASTM D2084 | (min) |

In den Anwendungs-Beispielen werden folgende Namen und Abkürzungen benutzt, deren Bedeutung im folgenden angegeben wird.

| | |
|---|---|
| RSS: | Ribbed Smoked Sheet (Naturkautschuk) |
| CORAX® N 220: | Ruß, Oberfläche (BET) 120 $m^2/g$ (Degussa) |
| Naftolen® ZD: | Weichmacher aus Kohlenwasserstoffen |
| Ingraplast R NS: | Weichmacher aus naphtenischen Kohlenwasserstoffen |
| Vulkanox® 3010 NA: | N-Isopropyl-N'-phenyl-p-phenylendiamin |
| Vulkanox® HS: | Poly-2,2,4-trimethyl-1,2-dihydrochinolin |
| Mesamoll®: | Alkylsulfonsäureester von Phenol und Kresol |
| Robac P 25 | Dipentamethylenthiuramtetrasulfid |
| Tetrone A | Dipentamethylenthiuramhexasulfid |
| Protektor® G 35: | Ozonschutzwachs |
| Vulkacit® MOZ: | Benzthiazolyl-2-morpholino-sulfenamid |

| | |
|---|---|
| Vulkacit® Mercapto: | 2-Mercaptobenzthiazol |
| Vulkacit® Thiuram: | Tetramethyl-thiurammono-sulfid |
| Vulkacit® CZ: | N-Cyclohexyl-2-benzothia-zolsulfenamid |
| Vulkalent® E: | N-Phenyl-N-(trichlor-methylsulfenyl)-benzol-sulfonamid |
| PVI: | N-Cyclohexylthiophthalimid |
| Ultrasil®VN 3: | gefällte Kieselsäure (Degussa) |
| Gran. | Granulat |
| V 143: | Bis-(2-Ethylamino-4-di-ethylamino-s-triazin-6-yl)-disulfid |
| Vulcacit® NZ | Benzothiazyl-2-tert.-butyl-sulfenamid |

Beispiel 7: Reversionsstabilität von mit N,N'-substituierten Bis-(2,4-diamino-s-triazin-6-yl) tetrasulfiden vernetztem, N 220-gefülltem NR (ohne Schwefel)

| | 1 | 2 | 3 | 4 |
|---|---|---|---|---|
| RSS 1, ML 4=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit MOZ | 1,43 | - | - | - |
| V 143 | - | 1,29 | - | - |
| Santoguard PVI | - | 0,4 | - | - |
| B | - | - | 3,34 | - |
| D | - | - | - | 4,10 |
| Schwefel | 1,5 | 1,5 | - | - |
| Reversion: | | | | |
| $\frac{D_{max} - D_{(max+60')}}{D_{max} - D_{min}}$ bei 170°C Vulkanisationstemperatur | 30,1 | 8,6 | 2,3 | 2,1 |

Fortsetzung

Beispiel 7: Reversionsstabilität von mit N,N'-substituierten Bis-(2,4-diamino-s-triazin-6-yl)-oligosulfiden vernetztem, N 220-gefülltem NR (ohne Schwefel)

| | 5 | 6 | 7 |
|---|---|---|---|
| RSS 1, ML 4=70-80 | 100 | 100 | 100 |
| CORAX N 220 | 50 | 50 | 50 |
| ZnO RS | 5 | 5 | 5 |
| Stearinsäure | 2 | 2 | 2 |
| Naftolen Z'D | 3 | 3 | 3 |
| Vulkanox 4010 NA | 2,5 | 2,5 | 2,5 |
| Vulkanox HS | 1,5 | 1,5 | 1,5 |
| Protector G 35 | 1 | 1 | 1 |
| K | 3,84 | - | - |
| L | - | 3,62 | - |
| M | - | - | 4,0 |
| Reversion: $\frac{D_{max} - D_{(max+60')}}{D_{max} - D_{min}}$ (%) bei 170°C Vulkanisationstemperatur | 2,5 | 3,1 | 2,3 |

Die erfindungsgemäßen N,N'-substituierten Bis-(2,4-diamino -s-triazin-6-yl)-oligosulfide erweisen sich beim Einsatz als Vernetzer (ohne Schwefel) als äußerst reversionsstabil in rußgefüllten NR-Mischungen (Mischungen 3-7) im Vergleich zu einem semi-EV-System (Mischung 1) beziehungsweise gegenüber einer Bis-(2-ethylamin-4-diethylamin-s-triazin-6-yl)-disulfid (V 143) haltigen Mischung (Mischung 2).

Beispiel 8: Reversionsstabilität von mit N,N'-substituierten Bis-(2,4-diamino-s-triazin-6-yl) tetrasulfiden vernetztem N 220/ kieselsäuregefüllten NR (Ohne Schwefel)

| | 8 | 9 | 10 | 11 | 12 |
|---|---|---|---|---|---|
| RSS 1, ML (1+4)=70-80 | 100 | 100 | 100 | 100 | 100 |
| CORAX N 220 | 25 | 25 | 25 | 25 | 25 |
| Ultrasil VN 3 Gran. | 25 | 25 | 25 | 25 | 25 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| C | - | 3,48 | - | - | - |
| E | - | - | 3,46 | - | - |
| G | - | - | - | 3,62 | - |
| I | - | - | - | - | 3,78 |
| Schwefel | 1,5 | - | - | - | - |
| Reversion: | | | | | |
| $\frac{D_{max} - D_{(max+60')}}{D_{max} - D_{min}}$ (%) bei 170°C Vulkanisationstemperatur | 47,1 | 1,8 | 2,4 | 3,3 | 6,2 |

Fortsetzung

Beispiel 8: Reversionsstabilität von mit N,N'-substituierten Bis-(2,4-diamino-s-triazin-6-yl)-oligosulfiden vernetztem N 220/ kieselsäuregefüllten NR (ohne Schwefel)

| | 13 | 14 | 15 |
|---|---|---|---|
| RSS 1, ML (1+4)=70-80 | 100 | 100 | 100 |
| CORAX N 220 | 25 | 25 | 25 |
| Ultrasil VN 3 Gran. | 25 | 25 | 25 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protector G 35 | 1 | 1 | 1 |
| K | 3,48 | - | - |
| L | - | 3,62 | - |
| M | - | - | 3,0 |
| Reversion: | | | |
| $\frac{D_{max} - D_{(max+60')}}{D_{max} - D_{min}}$ (%) bei 170°C Vulkanisationstemperatur | 2,5 | 3,1 | 4,7 |

Kieselsäurehaltige NR-Mischungen zeigen besonders starke Reversionserscheinungen auch bei Verwendung von semi-EV-Systemen (Mischung 8). Mit den erfindungsgemäß als Vernetzer eingesetzten N,N'-substituierten Bis-(2,4-diamino-s-triazin-6-yl)tetra- bzw. -oligosulfiden (Mischung 9 - 15) wird bei sonst gleicher Mischungszusammensetzung ein fast reversionsloser Zustand erreicht.

Beispiel 9: Reversionsbeständigkeit von mit N,N'-substituierten Bis-(2,4-diamino-s-triazin-6-yl)-tetrasulfiden beschleunigtem N 220-gefülltem NR

| | 16 | 17 | 18 | 19 | 20 |
|---|---|---|---|---|---|
| RSS 1, ML (1+4)=70-80 | 100 | 100 | 100 | 100 | 100 |
| CORAX N 220 | 50 | 50 | 50 | 50 | 50 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| B | - | 1,66 | - | - | - |
| C | - | - | 1,74 | - | - |
| D | - | - | - | 1,76 | - |
| E | - | - | - | - | 1,73 |
| Schwefel | 1,5 | 0,8 | 0,8 | 0,8 | 0,8 |
| Reversion: | | | | | |
| $\frac{D_{max} - D_{(max+60')}}{D_{max} - D_{min}}$ (%) bei 170°C Vulkanisationstemperatur | 31,9 | 0,4 | 0,0 | 0,0 | 1,3 |
| Vulkanisatdaten bei 170°C, t 95% | | | | | |
| Zugfestigkeit | 23,0 | 22,6 | 24,1 | 21,3 | 20,8 |
| Spannungswert 300% | 9,6 | 10,9 | 10,9 | 10,5 | 9,1 |

Fortsetzung (1)

Beispiel 9: Reversionsbeständigkeit von mit N,N'-substituierten Bis-(2,4-diamino-s-triazin-6-yl-)-tetrasulfiden beschleunigtem N 220-gefülltem NR

| | 21 | 22 | 23 | 24 |
|---|---|---|---|---|
| RSS 1, ML (1+4)=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| F | 1,65 | - | - | - |
| G | - | 1,81 | - | - |
| H | - | - | 1,82 | - |
| I | - | - | - | 1,82 |
| Schwefel | 0,8 | 0,8 | 0,8 | 0,8 |
| Reversion: | | | | |
| $\frac{D_{max} - D_{(max+60')}}{D_{max} - D_{min}}$ (%) bei 170°C Vulkanisationstemperatur | 1,3 | 2,0 | 1,2 | 2,1 |
| Vulkanisatdaten bei 170°C, t 95% | | | | |
| Zugfestigkeit | 19,9 | 22,1 | 22,0 | 23,2 |
| Spannungswert 300% | 10,4 | 10,8 | 11,9 | 10,5 |

Fortsetzung (2)

Beispiel 9: Reversionsbeständigkeit von mit N,N'-substituierten Bis-(2,4-diamino-s-triazin-6-yl)-oligosulfiden beschleunigtem N 220-gefülltem NR

| | 25 | 26 | 27 |
|---|---|---|---|
| RSS 1, (ML 1+4)=70-80 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protector G 35 | 1 | 1 | 1 |
| K | 1,74 | - | - |
| L | - | 1,81 | - |
| M | - | - | 1,5 |
| Schwefel | 0,8 | 0,8 | 0,8 |
| Reversion: | | | |
| $\frac{D_{max} - D_{(max+60')}}{D_{max} - D_{min}}$ (%) | 0,4 | 1,5 | 5,2 |
| bei 170°C Vulkanisationstemperatur | | | |
| Vulkanisatdaten bei 170°C, t 95% | | | |
| Zugfestigkeit | 22,9 | 24,2 | 22,2 |
| Spannungswert 300% | 10,3 | 10,0 | 10,6 |

Bei äquimolarer Beschleunigerdosierung kann im Fall der N,N'-substituierten Bis-(2,4-diamino-s-triazin-6-yl)tetra-beziehungsweise -oligosulfide die Schwefelmenge reduziert werden. Trotzdem werden höhere 300%-Spannungswerte erreicht. Die mit N,N'-substituierten Bis-(2,4-diamino-s-triazin-6-yl-)tetra- beziehungsweise oligosulfiden hergestellten Mischungen (Mischungen 17 - 27) erweisen sich als äußerst reversionsfest gegen ein semi-EV-System (Mischung 16 ).

Beispiel 10: Wirksamkeitsvergleich von Sulfenamid-mit N,N'-substituiertem Bis-(2,4-diamino-s-triazin-6-yl)-tetrasulfid-Beschleunigung in N 220-gefülltem NR bei gleicher $S_8$-Dosierung

| | 28 | 29 |
|---|---|---|
| RSS 1 ML (1+4)=70-80 | 100 | 100 |
| CORAX N 220 | 50 | 50 |
| ZnO RS | 5 | 5 |
| Stearinsäure | 2 | 2 |
| Naftolen ZD | 3 | 3 |
| Protector G 35 | 1 | 1 |
| Vulkanox 4010 NA | 2,5 | 2,5 |
| Vulkanox HS | 1,5 | 1,5 |
| Vulkacit MOZ | 1,43 | - |
| D | - | 1,76 |
| Schwefel | 1,5 | 1,5 |
| Reversion: | | |
| $\frac{D_{max} - D_{(max+60')}}{D_{max} - D_{min}}$ (%) bei 170°C Vulkanisationstemperatur | 31,9 | 5,1 |
| Vulkanisatdaten bei 160°C, t 95% | | |
| Spannungswert 300%, MPa | 10,6 | 12,8 |
| Shore-A-Härte | 63 | 64 |

Beispiel 9 zeigte den Vergleich von MOZ gegen N,N'-substituierte Bis-(2,4-diamino-s-triazin-6-yl)-tetrasulfide beziehungsweise deren Disproportionierungsprodukte bei unterschiedlicher Schwefelkonzentration. Hält man dagegen die Schwefelkonzentration konstant bei äquimolarer Beschleunigermenge , so bleibt der Reversionsunterschied erhalten, aber der Spannungswert 300% steigt beträchtlich an.

Beispiel 11: Vergleich des Reversionsverhaltens handelsüblicher Oligosulfide und Dibenzthiazolyl-tetrasulfid gegen N,N'-substituiertes Bis-(2,4-diamino-s-triazin-6-yl)- oligosulfid in N 220-gefülltem NR.

| | 30 | 31 | 32 | 33 |
|---|---|---|---|---|
| RSS 1, ML (1+4)=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Robac P 25 | 1,12 | - | - | - |
| Tetrone A | - | 1,3 | - | - |
| Dibenzthiazolyltetra-sulfid | - | - | 1,15 | - |
| M | - | - | - | 1,5 |
| Schwefel | 0,8 | 0,8 | 0,8 | 0,8 |
| Scorchzeit 170°C (t 10%), min | 1,4 | 1,9 | 2,7 | 3,1 |
| Reversion: $\frac{D_{max} - D_{(max+60')}}{D_{max} - D_{min}}$ (%) bei 170°C Vulkanisationstemperatur | 18,5 | 21,3 | 35,7 | 3,2 |
| Vulkanisatdaten bei 170°C, t 95 % | | | | |
| Zugfestigkeit | 23,1 | 21,2 | 18,9 | 23,6 |
| Spannungswert 300% | 9,4 | 9,5 | 7,3 | 10,4 |

Handelsübliche Oligosulfide (Mischung 30 und 31) wie auch Dibenzthiazolyltetrasulfid (Mischung 32) zeigen in NR mehrfach höhere Reversion als ein N,N'-substituiertes Bis-(2,4-diamino-s-triazin-6-yl-)-oligosulfid (Mischung 33).

Beispiel 12: Verschnitt von N.N'-substituiertem Bis-2,4-diamino-s-triazin-6-yl-)-tetrasulfid mit Schwefelspender in N 220-gefülltem NR

| | 34 | 35 |
|---|---|---|
| RSS 1 ML (1+4)=70-80 | 100 | 100 |
| CORAX N 220 | 50 | 50 |
| ZnO RS | 5 | 5 |
| Stearinsäure | 2 | 2 |
| Naftolen ZD | 3 | 3 |
| Protector G 35 | 1 | 1 |
| Vulkanox 4010 NA | 2,5 | 2,5 |
| Vulkanox HS | 1,5 | 1,5 |
| Vulkacit MOZ | 1,43 | - |
| Sulfasan R | - | 0,7 |
| D | - | 1,76 |
| Schwefel | 1,5 | - |
| Reversion: | | |
| $\frac{D_{max} - D_{(max+60')}}{D_{max} - D_{min}}$ (%) bei 170°C Vulkanisationstemperatur | 30,6 | 2,1 |

Gegen konventionelle Sulfenamidbeschleunigung (Mischung 34) ergibt sich bei Einsatz von N,N'-substituiertem Bis-(2,4-diamino-s-triazin-6-yl-)tetrasulfid mit Sulfasan R als Schwefelspender (Mischung 35 ) eine eindeutige Reversionsverbesserung.

Beispiel 13: Verschnitt von handelsüblichen Beschleunigern mit N,N'-substituiertem Bis-(2,4-diamino-s-triazin-6-yl)-tetrasulfid in N 220-gefülltem NR

| | 36 | 37 | 38 | 39 | 40 |
|---|---|---|---|---|---|
| RSS 1, ML (1+4)=70-80 | 100 | 100 | 100 | 100 | 100 |
| CORAX N 220 | 50 | 50 | 50 | 50 | 50 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | 0,71 | 1,5 | - | - |
| Vulkacit DM | - | - | - | 1,56 | 0,78 |
| B | - | 0,8 | 1,58 | - | 0,8 |
| Schwefel | 1,5 | 1,5 | - | 1,5 | 1,5 |
| Reversion: $\frac{D_{max} - D_{(max+60')}}{D_{max} - D_{min}}$ (%) bei 170°C Vulkanisationstemperatur | 28,7 | 16,8 | 5,7 | 31,2 | 16.1 |
| Vulkanisatdaten bei 170°C | | | | | |
| Spannungswert 300%, t 95% | 11,3 | 11,9 | 6,8 | 9,0 | 11,4 |
| t 95% + 90' | 8,9 | 10,7 | 6,7 | 7,5 | 10,5 |

Fortsetzung

Beispiel 13: Verschnitt von handelsüblichen Beschleunigern mit N,N'-substituiertem Bis-(2,4-diamino-s-triazin-6-yl-)-tetrasulfid in N 220-gefülltem NR

| | 41 | 42 | 43 | 44 |
|---|---|---|---|---|
| RSS 1, (ML 1+4)=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit DM | 1,56 | - | - | - |
| Vulkacit Merkapto | - | 1,2 | 0,6 | 1,2 |
| B | 1,58 | - | 0,8 | 1,58 |
| Schwefel | - | 1,5 | 1,5 | - |
| Reversion: $\frac{D_{max} - D_{(max+60')}}{D_{max} - D_{min}}$ (%) bei 170°C Vulkanisationstemperatur | 4,1 | 28,7 | 14,9 | 4,5 |
| Vulkanisatdaten bei 170°C | | | | |
| Spannungswert 300% t 95% | 7,0 | 7,8 | 11,3 | 5,9 |
| t 95% + 80' | 6,9 | 6,5 | 10,8 | 5,9 |

Die gemeinsame Verwendung von handelsüblichen Beschleunigern mit N,N'-substituierten Bis-(2,4-diamino-s-triazin-6-yl-) -tetrasulfiden (Mischungen 37, 40, 41) führt zu einer klaren Verbesserung des Reversionsverhaltens gegenüber der alleinigen Verwendung der handelsüblichen Beschleuniger (Mischungen 36, 39, 42). Eine weitere Erhöhung der Reversionsbeständigkeit läßt sich erzielen, indem der Schwefel eliminiert wird (Mischungen 38, 41, 44).

Beispiel 14: Verschnitt von N,N'-substituiertem Bis-(2,4-diamino-s-triazin-6-yl-)-tetrasulfid mit N,N'-substituiertem Bis-(2,4-diamino-s-triazin-6-yl)-oligosulfid in N 220-gefülltem NR.

| | 45 | 46 | 47 | 48 |
|---|---|---|---|---|
| RSS 1, ML (1+4)=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit MOZ | 1,43 | - | - | - |
| M | | 1,5 | - | 0,75 |
| B | - | - | 1,66 | 0,83 |
| Schwefel | 1,5 | 0,8 | 0,8 | 0,8 |
| Reversion: | | | | |
| $\frac{D_{max} - D_{(max+60')}}{D_{max} - D_{min}}$ (%) bei 170°C Vulkanisationstemperatur | 30,1 | 2,3 | 0,4 | 1,2 |

Durch die gemeinsame Verwendung von N,N'-substituierten Bis-(2,4-diamino-s-triazin-6-yl)-tetrasulfiden mit unterschiedlicher Reversionsbeständigkeit ergibt sich ein Wertebild, das zwischen den Reinsubstanzen liegt (Mischung 48).

Beispiel 15: Reversion bei Beschleunigung mit B in NR mit Ruß/Kieselsäure-Verschnitt.

| | 49 | 50 |
|---|---|---|
| RSS 1 ML (1+4)=70-80 | 100 | 100 |
| CORAX N 220 | 25 | 25 |
| Ultrasil VN 3 Gran. | 25 | 25 |
| ZnO RS | 5 | 5 |
| Stearinsäure | 2 | 2 |
| Naftolen D | 3 | 3 |
| Protector G 35 | 1 | 1 |
| Vulkanox 4010 NA | 2,5 | 2,5 |
| Vulkanox HS | 1,5 | 1,5 |
| Vulkacit MOZ | 1,43 | - |
| B | - | 1,67 |
| Schwefel | 1,5 | 0,8 |
| | | |
| Reversion: $\frac{D_{max} - D_{(max+60')}}{D_{max} - D_{min}}$ (%) bei 170°C Vulkanisationstemperatur | 46,7 | 10,8 |

Bei Ersatz des Sulfenamidbeschleunigers (Mischung 49) durch N,N'-substituiertes Bis-(2,4-diamino-s-triazin-6-yl-)tetrasulfid (Mischung 50) fällt die Reversion drastisch zurück.

Beispiel 16: N,N'-substituierte Bis-(2,4-diamino-s-triazin-6-yl)-tetra- und -oligosulfide in N 220-gefülltem SBR

| | 51 | 52 | 53 | 54 | 55 |
|---|---|---|---|---|---|
| SBR 1500 | 100 | 100 | 100 | 100 | 100 |
| CORAX N 220 | 50 | 50 | 50 | 50 | 50 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Protector G 35 | 1 | 1 | 1 | 1 | 1 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| B | - | 1,66 | - | - | - |
| F | - | - | 1,58 | - | - |
| K | - | - | - | 1,5 | - |
| M | - | - | - | - | 1,5 |
| Schwefel | 1,5 | 1,5 | 1,5 | 1,5 | 1,5 |
| Reversion: $\frac{D_{max} - D_{(max+60')}}{D_{max} - D_{min}}$ (%) bei 170°C Vulkanisationstemperatur | 12,9 | 11,3 | 11,5 | 9,2 | 12,5 |
| Vulkanisationsdaten bei 170°C, t 95 % | | | | | |
| Zugfestigkeit | 20,6 | 21,3 | 20,8 | 20,5 | 19,9 |
| Spannungswert 300% | 10,0 | 11,6 | 12,2 | 12,1 | 12,3 |
| Bruchdehnung | 460 | 440 | 420 | 420 | 390 |
| Shore-Härte | 61 | 63 | 63 | 63 | 64 |

Bei gleicher Schwefeldosierung zeigen N,N'-substituierte Bis-(2,4-diamino-s-triazin-6-yl-)-tetra- beziehungsweise -oligosulfide (Mischung 52 - 55) in SBR 1500 einen deutlich höheren Spannungswert und leicht verbesserte Reversionseigenschaften gegen eine konventionelle Beschleunigung (Mischung 51).

Beispiel 17: N,N'-substituierte Bis-(2,4-diamino-s-triazin-6-yl-)-tetra- bzw.-oligosulfide in N 220-gefülltem Isoprenkautschuk

| | 56 | 57 | 58 | 59 |
|---|---|---|---|---|
| Polyisopren 3,4 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit MOZ | 1,43 | - | - | - |
| B | - | 1,76 | - | - |
| L | - | - | 1,6 | - |
| M | - | - | - | 1,5 |
| Schwefel | 1,5 | 0,8 | 0,8 | 0,8 |
| Reversion: | | | | |
| $\frac{D_{max} - D_{(max+60')}}{D_{max} - D_{min}}$ (%) beo 170°C Vulkanisationstemperatur | 21,0 | 2,1 | 0,0 | 0,0 |
| Vulkanisatdaten bei 170°C, | | | | |
| Spannungswert 300% t 95% | 7,9 | 6,6 | 6,3 | 6,4 |
| t 95% + 80' | 6,2 | 6,8 | 6,3 | 7,1 |

In Polyisoprenkautschuk wird durch den Einsatz von N,N'-substituierten Bis-(2,4-diamino-s-triazin-6-yl-)tetra-beziehungsweise -oligosulfiden (Mischungen 57 - 59) gegen konventionelle Beschleuniger (Mischung 56) ebenfalls eine deutliche Verbesserung der Reversion erreicht. Diese Verbesserung zeigt sich auch bei der Konstanz der Vulkanisatdaten bei deutlicher Überheizung.

Beispiel 18: N,N'-substituierte Bis-(2,4-diamino-triazin-6-yl)-tetra- und -oligosulfide in N 220-gefülltem EPDM

| | 60 | 61 | 62 |
|---|---|---|---|
| Buna AP 451 | 100 | 100 | 100 |
| CORAX N 220 | 50 | 50 | 50 |
| ZnO RS | 5 | 5 | 5 |
| Stearinsäure | 2 | 2 | 2 |
| Ingraplast NS | 10 | 10 | 10 |
| Vulkacit Thiuram | 1 | - | - |
| Vulkacit Mercapto | 0,5 | - | - |
| B | - | 2,93 | - |
| M | - | - | 2,5 |
| Schwefel | 1 | 1 | 1 |
| $\frac{D_{max} - D_{(max+60')}}{D_{max} - D_{min}}$ (%) bei 160°C Vulkanisationstemperatur | 3,3 | 0,0 | 0,0 |

Bereits hochreversionsfeste EPDM-Mischungen zeigen eine weitere Reduktion der Reversion auf Null, wenn konventionelle Beschleunigergemische (Mischung 60) durch N,N'-substituierte Bis-(2,4-diamino-s-triazin-6-yl)tetra-beziehungsweise -oligosulfide ersetzt werden (Mischungen 61 und 62).

Beispiel 19: N,N'-substituierte Bis-(2,4-diamino-s-triazin-6-yl-)-tetra und -oligosulfide in N 220-gefülltem NBR

| | 63 | 64 | 65 |
|---|---|---|---|
| Perbunan N 3307 NS | 100 | 100 | 100 |
| CORAX N 220 | 60 | 60 | 60 |
| ZnO RS | 5 | 5 | 5 |
| Stearinsäure | 1 | 1 | 1 |
| Paraffin fest | 1 | 1 | 1 |
| Mesamoll | 10 | 10 | 10 |
| Vulkanox HS | 1,5 | 1,5 | 1,5 |
| Vulkacit CZ | 1,5 | 1,5 | 1,5 |
| D | - | 1,76 | - |
| M | - | - | 1,5 |
| Schwefel | 1,2 | 1,2 | 1,2 |
| Reversion: $\frac{D_{max} - D_{(max+60')}}{D_{max} - D_{min}}$ (%) bei 170°C Vulkanisationstemperatur | 8,0 | 5,4 | 5,6 |
| Vulkanisatdaten bei 170°C, t 95% | | | |
| Spannungswert 300% | 11,8 | 14,0 | 14,6 |
| Shore-Härte | 68 | 69 | 69 |

Bei gleichem Schwefelgehalt tritt gegen die Referenzmischung (Mischung 63) bei Ersatz durch N,N'-substituierte Bis-(2,4-diamino-s-triazin-6-yl-)-tetra- beziehungsweise -oligosulfide (Mischungen 64 und 65) in NBR eine kräftige Steigerung des Spannungswertes bei 300% Dehnung bei gleichzeitiger Reduktion der Reversion ein.

Beispiel 20: Reversionsstabilität N,N'-substituierter Bis-(2,4-diamino-s-triazin-6-yl-) -oligo sulfide in N 220-gefülltem BR

| | 66 | 67 | 68 |
|---|---|---|---|
| Buna CB 10 | 100 | 100 | 100 |
| CORAX N 220 | 60 | 60 | 60 |
| ZnO RS | 3 | 3 | 3 |
| Stearinsäure | 2 | 2 | 2 |
| Naftolen ZD | 15 | 15 | 15 |
| Protector G 35 | 1 | 1 | 1 |
| Vulkanox 4010 NA | 1,5 | 1,5 | 1,5 |
| Vulkacit NZ | 1,5 | - | - |
| M | - | 1,5 | - |
| D | - | - | 1,76 |
| Schwefel | 1,5 | 1,5 | 1,5 |
| Reversion: | | | |
| $\frac{D_{max} - D_{(max+60')}}{D_{max} - D_{min}}$ (%) bei 170°C Vulkanisationstemperatur | 30,3 | 20,8 | 19,1 |
| Vulkanisatdaten bei 170°C | | | |
| Spannungswert 300% t 95% | 8,6 | 8,9 | 8,2 |
| t 95% + 75' | 5,6 | 6,8 | 6,6 |

Wegen der geringeren Reversion der M und D beschleunigten BR-Mischungen (Mischungen 67 und 68) ist der Abfall im Spannungswert 300% bei starker Überheizung deutlich gegen die Referenzmischung (Mischung 66) verringert.

Beispiel 21: N,N'-substituierte Bis-(2,4-diamino-s-triazin-6-yl-)-tetra- und -oligosulfide in N 220-gefülltem NR/BR-Verschnitt

| | 69 | 70 | 71 | 72 |
|---|---|---|---|---|
| RSS 1, ML (1+4)=70-80 | 70 | 70 | 70 | 70 |
| Buna CB 10 | 30 | 30 | 30 | 30 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit MOZ | 1,43 | - | - | - |
| C | - | 1,74 | - | - |
| D | - | - | 1,76 | - |
| M | - | - | - | 1,5 |
| Schwefel | 1,5 | 0,8 | 0,8 | 0,8 |
| Reversion: | | | | |
| $\frac{D_{max} - D_{(max+60')}}{D_{max} - D_{min}}$ (%) bei 170°C Vulkanisationstemperatur | 32,0 | 15,8 | 20,6 | 23,8 |

Die Mischungen 70 bis 72 zeigen eine deutliche Verringerung der Reversion bei Einsatz von N,N'-substituierten Bis-(2,4-diamino-s-triazin-6-yl-)-tetra- und-oligosulfiden gegen ein semi-EV-System (Mischung 69) in einem Verschnitt von NR und Polybutadien.

Beispiel 22: Vernetzung von zu 50% epoxidiertem Natur kautschuk (ENR 50) bei N 220-füllung mit N, N'-substituierte Bis-(2,4-diamino-s-triazin-6-yl-)tetra- bzw.oligosulfid

| | 73 | 74 | 75 |
|---|---|---|---|
| ENR 50 | 100 | 100 | 100 |
| CORAX N 220 | 50 | 50 | 50 |
| ZnO RS | 5 | 5 | 5 |
| Stearinsäure | 2 | 2 | 2 |
| Vulkanox HS | 2 | 2 | 2 |
| Vulkacit MOZ | 2,4 | - | - |
| Vulkacit Thiuram | 1,6 | - | - |
| C | - | 4,6 | - |
| M | - | - | 4,0 |
| Schwefel | 0,3 | 0,3 | 0,3 |
| Reversion: | | | |
| $\frac{D_{max} - D_{(max+60')}}{D_{max} - D_{min}}$ (%) bei 150°C Vulkanisationstemperatur | 6,7 | 0,0 | 0,0 |
| Vulkanisatdaten bei 150°C, t 95% | | | |
| Zugfestigkeit | 18,7 | 23,9 | 24,6 |
| Spannungswert 300 % | 18,0 | 18,4 | 18,9 |
| Weiterreißwiderstand | 12 | 14 | 12 |
| Shore-Härte | 75 | 76 | 78 |

Bei gleichem Spannungswert bei 300% Dehnung und beträchtlich gesteigerter Zerreißfestigkeit führt der Ersatz einer konventionellen Beschleunigung (Mischung 73) durch N,N'-substituierte Bis-(2,4-diamino-s-triazin-6-yl-)-tetra- beziehungsweise -oligosulfide (Mischungen 74 und 75) zu einer zusätzlichen Verbesserung der Reversion.

Beispiel 23: Wirkung von Vulkalent E bei schwefelfreier NR-Vernetzung von N 220- und N 220/Kieselsäure-gefülltem NR mit N,N'-substituierten Bis-(2,4-diamino-s-triazin-6-yl-)-tetrasulfiden

| | 76 | 77 | 78 | 79 | 80 | 81 |
|---|---|---|---|---|---|---|
| RSS 1, ML (1+4)=70-80 | 100 | 100 | 100 | 100 | 100 | 100 |
| CORAX N 220 | 50 | 50 | 50 | 25 | 25 | 25 |
| Ultrasil VN 3 Gran. | - | - | - | 25 | 25 | 25 |
| ZnO RS | 5 | 5 | 5 | 5 | 5 | 5 |
| Stearinsäure | 2 | 2 | 2 | 2 | 2 | 2 |
| Naftolen ZD | 3 | 3 | 3 | 3 | 3 | 3 |
| Vulkanox 4010 NA | 2,5 | 2,5 | 2,5 | 2,5 | 2,5 | 2,5 |
| Vulkanox HS | 1,5 | 1,5 | 1,5 | 1,5 | 1,5 | 1,5 |
| Protector G 35 | 1 | 1 | 1 | 1 | 1 | 1 |
| Vulkacit MOZ | 1,43 | - | - | 1,43 | - | - |
| C | - | 3,79 | 3,79 | - | 2,84 | 2,84 |
| Vulkalent E | - | - | 3,2 | - | - | 2,4 |
| Schwefel | 1,5 | - | - | 1,5 | - | - |
| Reversion: | | | | | | |
| $\frac{D_{max} - D_{(max+60')}}{D_{max} - D_{min}}$ (%) bei 170°C Vulkanisationstemperatur | 32,5 | 4,1 | 4,5 | 47,8 | 5,4 | 5,5 |
| ti 170°C ,(min) | 4,2 | 2,8 | 4,6 | 4,7 | 3,0 | 4,7 |

In N 220 und N 220/Kieselsäure gefülltem und mit C vernetztem NR erreicht man durch den Vulkalent E-Zusatz die gewünschte Verlängerung der Inkubationszeit, ohne daß dabei die Reversion beeinträchtigt wird.

Beispiel 24: Verzögernde Wirkung von Vulkalent E auf N,N'-substituierte Bis-(2,4-diamino-triazin-6-yl-)tetra- und -oligosulfid vulkanisierte N 220/ NR-Mischungen

| | 82 | 83 | 84 | 85 | 86 |
|---|---|---|---|---|---|
| RSS 1, ML 4 = 70-80 | 100 | 100 | 100 | 100 | 100 |
| CORAX N 220 | 50 | 50 | 50 | 50 | 50 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| B | - | 1,66 | 1,66 | - | - |
| M | - | - | - | 1,55 | 1,55 |
| Schwefel | 1,5 | 0,8 | 0,8 | 0,8 | 0,8 |
| Vulkalent E | - | - | 1,2 | - | 1,2 |
| ti (min bei 170 °C | 3,8 | 3,0 | 4,4 | 2,9 | 4,1 |
| Vulkanisatdaten bei 170 °C, t 95% | | | | | |
| Spannungswert 300% | 10,6 | 10,2 | 11,5 | 10,6 | 11,0 |

Der Zusatz von Vulkalent E in B und M beschleunigten Mischungen (Mischungen 84 und 86) führt zu einer Anhebung der Inkubationszeit ti über das Niveau der konventionell mit MOZ beschleunigten Mischung (Mischung 82).

Beispiel 25: N,N'-substituierte Bis-(2,4-diamino-s-triazin-6-yl-)-tetrasulfide mit Vulkalent-E-Zusatz in N 220-gefülltem NR in Abhängigkeit von der Temperatur

| | 87 | 88 | 89 |
|---|---|---|---|
| RSS 1 ML (1+4)=70-80 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Protector G 35 | 1 | 1 | 1 |
| Vulkanox 4010 NA | 2,5 | 2,5 | 2,5 |
| Vulkanox HS | 1,5 | 1,5 | 1,5 |
| Vulkacit MOZ | 1,43 | - | - |
| B | - | 1,66 | 1,66 |
| Vulkalent E | - | - | 1,2 |
| Schwefel | 1,5 | 0,8 | 0,8 |

Reversion:

$$\frac{D_{max} - D_{(max+60')}}{D_{max} - D_{min}} \ (\%)$$

| | 87 | 88 | 89 |
|---|---|---|---|
| bei 145°C Prüftemperatur | 7,9 | 0,0 | 0,0 |
| 160°C " | 25,6 | 0,0 | 1,9 |
| 170°C " | 30,9 | 0,4 | 3,6 |
| 180°C " | 39,1 | 2,6 | 5,5 |

Beispiel 25 zeigt bei reiner N,N'-substituierter Bis-(2,4-diamino-s-triazin-6-yl)-tetrasulfid-Beschleunigung (Mischung 88) wie auch bei Zusatz des Vulkalent E (Mischung 89) gegenüber konventioneller Beschleunigung (Mischung 87) eine ausgeprägte Temperaturunempfindlichkeit der Reversion.

Beispiel 26: Wirkung von Vulkalent E auf N,N'-substituierte Bis-(2,4-diamino-s-triazin-6-yl-) -tetrasulfid undoligosulfid vernetztem NR mit N 220/ Kieselsäure

| | 90 | 91 | 92 | 93 | 94 |
|---|---|---|---|---|---|
| RSS 1, ML (1 + 4) = 70-80 | 100 | 100 | 100 | 100 | 100 |
| Ultrasil VN 3 Gran. | 25 | 25 | 25 | 25 | 25 |
| CORAX N 220 | 25 | 25 | 25 | 25 | 25 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| D | - | 3,5 | 3,5 | - | - |
| M | - | - | - | 3,0 | 3,0 |
| Vulkalent E | - | - | 1,2 | - | 1,2 |
| Schwefel | 1,5 | 0,8 | 0,8 | 0,8 | 0,8 |
| Scorch bei 130 ° C (min) | 29,5 | 15,0 | 28,5 | 16,5 | 29,0 |
| Scorch bei 170 ° C (min) | 4,5 | 3,4 | 4,4 | 3,7 | 4,8 |

Mit Vulkalent E läßt sich bei Beschleunigung mit N,N'-substituierten Bis-(2,4-diamino-s-triazin-6-yl)-tetra-beziehungsweise -oligosulfiden (Mischungen 92 und 94) ein zu MOZ vergleichbares Scorchverhalten einstellen.

Beispiel 27: Gleichzeitige Verwendung von N,N'-substituiertem Bis-(2,4-diamino-s-triazin-6-yl-)-tetrasulfid und Si 69 mit beziehungsweise ohne Vulkalent E in N 220/VN 3-gefülltem NR

| | 95 | 96 | 97 | 98 | 99 |
|---|---|---|---|---|---|
| RSS 1, ML (1+4)=70-80 | 100 | 100 | 100 | 100 | 100 |
| CORAX N 220 | 25 | 25 | 25 | 25 | 25 |
| Ultrasil VN 3 Gran. | 25 | 25 | 25 | 25 | 25 |
| Si 69 | 3,75 | 3,75 | 3,75 | 3,75 | 3,75 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| B | - | 2,2 | 2,2 | 1,66 | 1,66 |
| Vulkalent E | - | - | 1,6 | - | 1,2 |
| Schwefel | 1,5 | - | - | 0,8 | 0,8 |
| Reversion: | | | | | |
| $\frac{D_{max} - D_{(max+60')}}{D_{max} - D_{min}}$ (%) bei 170°C Vulkanisationstemperatur | 19,6 | 0,0 | 0,0 | 0,0 | 0,0 |
| ti bei 170°C (min) | 4,6 | 4,2 | 5,7 | 4,2 | 5,2 |

Bei gemeinsamem Einsatz von N,N'-substituierten Bis-(2,4-diamino-s-triazin-6-yl-)-tetrasulfiden und Si 69 werden reversionsfreie Mischungen sowohl mit wie auch ohne Schwefel (Mischungen 96, 98) erhalten gegen konventionelle Beschleuniger mit Silan (Mischung 95). Der Zusatz von Vulkalent E führt auch in diesem Fall zu einer Verlängerung der Scorchzeit, ohne daß dabei die Reversion wieder auftritt (Mischungen 97 und 99).

Beispiel 28: Vernetzung von N 220/Kieselsäure-gefülltem NR mit N,N'-substituiertem Bis-(2,4-diamino-triazin-6-yl)-tetrasulfid ohne Schwefel

| | | | | | | | |
|---|---|---|---|---|---|---|---|
| RSS 1 | 100 | 100 | 100 | 100 | 100 | 100 | 100 |
| CORAX N 220 | 25 | 25 | 25 | 25 | 25 | 25 | 25 |
| Ultr. VN 3 Gran. | 25 | 25 | 25 | 25 | 25 | 25 | 25 |
| ZnO RS | 5 | 5 | 5 | 5 | 5 | 5 | 5 |
| Stearinsäure | 2 | 2 | 2 | 2 | 2 | 2 | 2 |
| Naftolen ZD | 3 | 3 | 3 | 3 | 3 | 3 | 3 |
| Vulkanox 4010 NA | 2,5 | 2,5 | 2,5 | 2,5 | 2,5 | 2,5 | 2,5 |
| Vulkanox HS | 1,5 | 1,5 | 1,5 | 1,5 | 1,5 | 1,5 | 1,5 |
| Protector G 35 | 1 | 1 | 1 | 1 | 1 | 1 | 1 |
| Vulkacit MOZ | 1,43 | - | - | - | - | - | - |
| D | - | 2 | 3 | 4 | 5 | 6 | 7 |
| Schwefel | 1,5 | - | - | - | - | - | - |
| $\frac{D_{max}-D_{(max+60')}}{D_{max}-D_{min}}$ % bei 170°C Vulkanisationstemperatur | 44,7 | 3,2 | 2,0 | 1,3 | 3,1 | 2,8 | 2,6 |
| Vulkanisatdaten (t 95%) bei 170°C | | | | | | | |
| Spannungswert 300% | 4,9 | 2,6 | 4,4 | 5,8 | 6,8 | 7,8 | 8,8 |

Die schwefelfreie Vernetzung mit N,N'-substituiertem Bis-(2,4-diamino-triazin-6-yl)-tetrasulfid führt in Naturkautschuk mit Ruß- und Kieselsäurefüllung zu einer Unterbindung der Reversion und gleichzeitig zu einer mengenabhängigen Spannungswerterhöhung.

Beispiel 29: Vernetzung von Kieselsäure-gefülltem NR mit N,N'-substituiertem Bis-(2,4-diamino-triazin-6-yl)-tetrasulfid

| | | |
|---|---|---|
| RSS 1 | 100 | 100 |
| Ultr. VN 3 Gran. | 50 | 50 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protector G 35 | 1 | 1 |
| Vulkacit MOZ | 1,43 | - |
| D | - | 7 |
| Schwefel | 1,5 | - |
| $\frac{D_{max} - D_{(max+60')}}{D_{max} - D_{min}}$ % bei 170° C Vulkanisationstemperatur | 33,0 | 7,8 |
| Vulkanisatdaten (t 95 %) bei 170° C | | |
| Spannungswert 300 % | 2,7 | 4,3 |

Die schwefelfreie Vernetzung mit N,N'-substituiertem Bis-(2,4-diamino-triazin-6-yl)-tetrasulfid führt in Naturkautschuk mit Kieselsäurefüllung zu einer weitgehenden Reduzierung der Reversion und gleichzeitig zu einer Erhöhung des Spannungswertes bei 300 %.

Beispiel 30: Beschleunigung von N 220/Kieselsäure-gefülltem NR mit N,N'-substituiertem Bis-(2,4-diamino-triazin-6-yl)-tetrasulfid

| | | | | | | | |
|---|---|---|---|---|---|---|---|
| RSS 1 | 100 | 100 | 100 | 100 | 100 | 100 | 100 |
| CORAX N 220 | 25 | 25 | 25 | 25 | 25 | 25 | 25 |
| Ultr. VN 3 Gran. | 25 | 25 | 25 | 25 | 25 | 25 | 25 |
| ZnO RS | 5 | 5 | 5 | 5 | 5 | 5 | 5 |
| Stearinsäure | 2 | 2 | 2 | 2 | 2 | 2 | 2 |
| Naftolen ZD | 3 | 3 | 3 | 3 | 3 | 3 | 3 |
| Vulkanox 4010 NA | 2,5 | 2,5 | 2,5 | 2,5 | 2,5 | 2,5 | 2,5 |
| Vulkanox HS | 1,5 | 1,5 | 1,5 | 1,5 | 1,5 | 1,5 | 1,5 |
| Protector G 35 | 1 | 1 | 1 | 1 | 1 | 1 | 1 |
| Vulkacit MOZ | 1,43 | - | - | - | - | - | - |
| B | - | 2 | 3 | 4 | 5 | 6 | 7 |
| Schwefel | 1,5 | 0,51 | 0,76 | 1,02 | 1,27 | 1,53 | 1,79 |
| $\frac{D_{max}-D_{(max+60')}}{D_{max}-D_{min}}$ % | 44,7 | 3,2 | 1,7 | 0 | 0 | 0,8 | 1,7 |
| bei 170°C Vulkanisationstemperatur | | | | | | | |
| Vulkanisatdaten (t 95%) bei 170°C | | | | | | | |
| Spannungswert 300% | 3,6 | 3,5 | 5,5 | 7,0 | 8,3 | 9,2 | 9,8 |

Die Schwefelvulkanisation mit N,N'-substituiertem Bis-(2,4-diamino-triazin-6-yl)-tetrasulfid von N 220/Kieselsäure-gefülltem NR reduziert die Reversion auf 0% und führt zu einer starken Steigerung des Spannungswertes.

Beispiel 31: Beschleunigung von Kieselsäure-gefülltem NR mit N,N'-substi-tuiertem Bis-(2,4-diamino-triazin-6-yl)-tetrasulfid

| | | |
|---|---|---|
| RSS 1 | 100 | 100 |
| Ultr. VN 3 Gran. | 50 | 50 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protector G 35 | 1 | 1 |
| Vulkacit MOZ | 1,43 | - |
| B | - | 7 |
| Schwefel | 1,5 | 1,79 |
| $\frac{D_{max} - D_{(max+60')}}{D_{max} - D_{min}}$ % | 33,0 | 4,5 |

bei 170° C Vulkanisationstemperatur

Vulkanisatdaten (t 95 %) bei 170° C

| | | |
|---|---|---|
| Spannungswert 300 % | 2,7 | 5,3 |

Die Schwefelvulkanisation mit N,N'-substituiertem Bis-(2,4-diamino-triazin-6-yl)-tetrasulfid von kieselsäure-gefülltem NR gelingt nahezu reversionsfrei und unter Steigerung des 300 %-Spannungswertes.

Beispiel 32: Beschleunigung von N 220/ Kieselsäure-gefülltem NR mit N,N'-substituiertem Bis-(2,4-diamino-triazin-6-yl)-tetrasulfid in Gegenwart von Si 69

| | | | |
|---|---|---|---|
| RSS | 100 | 100 | 100 |
| CORAX N 220 | 25 | 25 | 25 |
| Ultr. VN 3 Gran. | 25 | 25 | 25 |
| Si 69 | 3,75 | 3,75 | 3,75 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protector G 35 | 1 | 1 | 1 |
| Vulkacit MOZ | 1,43 | - | - |
| B | - | 2 | 3 |
| Schwefel | 1,5 | 0,49 | 0,74 |
| $\frac{D_{max} - D_{(max+60')}}{D_{max} - D_{min}}$ % | 18,1 | 0 | 0 |

bei 170° C Vulkanisationstemperatur

Vulkanisatdaten (t 95%) bei 170° C

| | | | |
|---|---|---|---|
| Spannungswert 100% | 1,4 | 1,6 | 2,4 |
| Spannungswert 200% | 3,5 | 4,6 | 7,3 |
| Spannungswert 300% | 7,1 | 9,5 | 13,9 |

Die Beschleunigung mit N,N'-substitiuertem Bis- (2,4-diamino-triazin-6-yl)-tetrasulfid in Gegenwart von Si 69 gestattet die Rückführung der Reversion auf O% bei gleichzeitiger kräftiger Erhöhung der Spannungswerte.

Beispiel 33: Beschleunigung von Kieselsäure-gefülltem NR mit N,N'-substituiertem Bis-(2,4-diamino-triazin-6-yl)-tetrasulfid in Gegenwart von Si 69

| | | | | |
|---|---|---|---|---|
| RSS 1 | 100 | 100 | 100 | 100 |
| Ultr. VN 3 Gran. | 50 | 50 | 50 | 50 |
| Si 69 | 7,5 | 7,5 | 7,5 | 7,5 |
| ZnO RS | 5 | 5 | 5 | 5 |
| Stearingsäure | 2 | 2 | 2 | 2 |
| Naftolen ZD | 3 | 3 | 3 | 3 |
| Vulkanox 4010 NA | 2,5 | 2,5 | 2,5 | 2,5 |
| Vulkanox HS | 1,5 | 1,5 | 1,5 | 1,5 |
| Protector G 35 | 1 | 1 | 1 | 1 |
| Vulkacit MOZ | 1,43 | - | - | - |
| B | - | 2 | 3 | 4 |
| Schwefel | 1,5 | 0,49 | 0,74 | 0,99 |

| | | | | |
|---|---|---|---|---|
| $\frac{D_{max}-D_{(max+60')}}{D_{max}-D_{min}}$ % | 16,6 | 0 | 0 | 0 |

bei 170° C Vulkanisationstemperatur

Vulkanisatdaten (t 95%) bei 170° C

| | | | | |
|---|---|---|---|---|
| Spannungswert 100% | 1,5 | 1,7 | 2,5 | 3,2 |
| Spannungswert 200% | 3,4 | 4,3 | 6,6 | 8,3 |
| Spanungswert 300% | 6,3 | 8,2 | 12,3 | 15,0 |

Auch im Falle reiner Krieselsäurefüllung wird NR in Gegenwart von Si 69 mit N,N'-substituiertem Bis-(2,4-diamino-triazin-6-yl)-tetradulfid und Schwefel reversionsfrei vulkanisiert, wobei gleichzeitig das Spannungswertniveau sehr stark angehoben wird.

Beispiel 34: Die schwefelfreie Vernetzung von N 220/Kieselsäure-gefülltem NR mit N,N'-substituiertem Bis-(2,4-diamino-triazin-6-yl)-tetrasulfid in Gegenwart von Si 69

| | | | | | |
|---|---|---|---|---|---|
| RSS 1 | 100 | 100 | 100 | 100 | 100 |
| CORAX N 220 | 25 | 25 | 25 | 25 | 25 |
| Ultr. VN 3 Gran. | 25 | 25 | 25 | 25 | 25 |
| Si 69 | 3,75 | 3,75 | 3,75 | 3,75 | 3,75 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| D | - | 1 | 2 | 3 | 4 |
| Schwefel | 1,5 | - | - | - | - |
| $\frac{D_{max}-D_{(max+60')}}{D_{max}-D_{min}}$ % | 18,1 | 0 | 0 | 0 | 0 |

bei 170°C Vulkanisationstemperatur

Vulkanisatdaten (t 95%) bei 170°C

| | | | | | |
|---|---|---|---|---|---|
| Spannungswert 200% | 3,5 | 2,6 | 4,9 | 6,5 | 8,6 |
| Spannungswert 300% | 7,1 | 5,8 | 10,2 | 12,9 | 16,0 |

Die schwefelfreie Vernetzung mit N,N'-substituiertem Bis-(2,4-diamino-triazin-6-yl)-tetrasulfid führt in Naturkautschuk mit Ruß- und Kieselsäurefüllung auch bei Zusatz von Si 69 zu einer gleichzeitigen Verhinderung der Reversion und einer kräftigen mengenanhängigen Spannungswerterhöhung.

Beispiel 35: Die schwefelfreie Vernetzung von Kieselsäure-gefülltem NR mit N,N'-substituiertem Bis-(2,4-diamino-triazin-6-yl)-tetrasulfid in Gegenwart von Si 69

| | | | | | |
|---|---|---|---|---|---|
| RSS 1 | 100 | 100 | 100 | 100 | 100 |
| Ultr.VN 3 Gran. | 50 | 50 | 50 | 50 | 50 |
| Si 69 | 7,5 | 7,5 | 7,5 | 7,5 | 7,5 |
| ZnO RS | 5 | 5 | 5 | 5 | 5 |
| Stearinsäure | 2 | 2 | 2 | 2 | 2 |
| Naftolen ZD | 3 | 3 | 3 | 3 | 3 |
| Vulkanox 4010 NA | 2,5 | 2,5 | 2,5 | 2,5 | 2,5 |
| Vulkanox HS | 1,5 | 1,5 | 1,5 | 1,5 | 1,5 |
| Protector G 35 | 1 | 1 | 1 | 1 | 1 |
| Vulkacit MOZ | 1,43 | - | - | - | - |
| D | - | 1 | 2 | 2 | 4 |
| Schwefel | 1,5 | - | - | - | - |
| $\frac{D_{max}-D_{(max+60')}}{D_{max}-D_{min}}$ % | 16,6 | 0 | 0 | 0 | 0 |

bei 170°C Vulkanisationstemperatur

Vulkanisatdaten (t 95%) bei 170°C

| | | | | | |
|---|---|---|---|---|---|
| Spannungswert 200% | 3,4 | 2,8 | 3,9 | 5,0 | 7,6 |
| Spannungswert 300% | 6,3 | 5,0 | 7,6 | 11,3 | 13,9 |

Die Vernetzung mit N,N'-substituiertem Bis-(2,4-diamino-triazin-6-yl)-tetrasulfid von Naturkautschuk mit reiner Kieselsäurefüllung in Anwesenheit von Si 69 gelingt ebenfalls reversionsfrei und führt gleichzeitig zur kräftigen Spannungswerterhöhung.

## Patentansprüche

**1.** Verbindungen der allgemeinen Formel

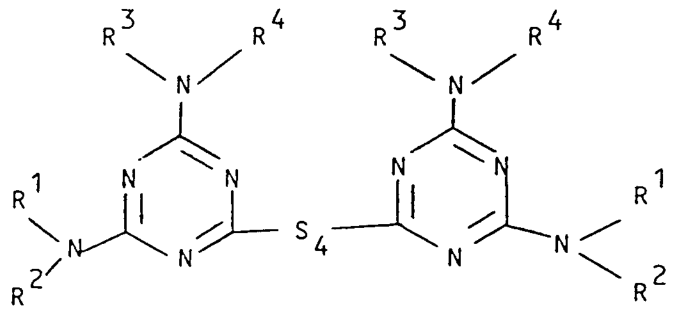

(I)

in welcher bedeuten:

$R^1$, $R^2$ = H, $R^2$ = Benzyl,

$R^2$, $R^3$, $R^4$ = $C_1$-$C_8$ -Alkyl, Allyl, $C_3$-$C_8$ Cycloalkyl, letzteres unsubstituiert oder mit 1-3 Methylgruppen substituiert, 2-Hydroxyethyl, 3-Hydroxypropyl, 2-Hydroxypropyl

mit Ausnahme des Bis-(2-Ethylamino-4-diethylamino-s-triazin-6-yl)tetrasulfids.

**2.** Gemische, bestehend aus Verbindungen der allgemeinen Formel

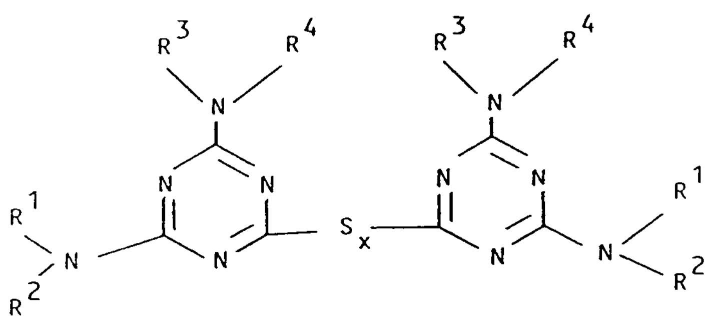

(II)

in der $R^1$, $R^2$, $R^3$ und$R^4$ dieselben Bedeutungen wie in Anspruch 1 haben und $S_x$ einer mittleren statistischen Kettenlange mit x = 4 entspricht, mit Ausnahme des Bis-(2-Ethylamino-4-diethylamino-s-triazin-6-yl)tetrasulfids.

**3.** Verfahren zur Herstellung der Tetrasulfide gemäß Anspruch 1, dadurch gekennzeichnet, daß man ein wässrige alkalische Lösung der entsprechenden N,N'-substituierten 2,4-Diamino-6-mercaptotriazine in einem Zweiphasensystem mit einer Lösung von $S_2Cl_2$ in einem inerten, das entstehende Tetrasulfid nicht oder wenig lösenden, organischen Lösungsmittel bei Temperaturen zwischen -5 °C und < +20 °C umsetzt, wobei sich das Verhältnis von Mercaptotriazin zu $S_2Cl_2$ auf 2 : 1 bis 2 : 1,2 beläuft.

**4.** Verfahren zur Herstellung der Tetrasulfide gemäß Anspruch 3, dadurch gekennzeichnet, daß man die mindestens zur Umsetzung notwendige stöchiometrische Menge an Alkalihydroxid verwendet.

**5.** Verfahren gemäß den Ansprüchen 3 bis 4, dadurch gekennzeichnet, daß man Natrium- oder Kaliumhydroxid und als Lösungsmittel, $C_5$-$C_{10}$ Alkane oder $C_5$-$C_8$ Cycloalkane, gegebenenfalls substituiert mit 1 - 3 Methylgruppen, verwendet.

**6.** Verfahren zur Herstellung von Gemischen bestehend aus Verbindungen gemäß Anspruch 2, dadurch gekennzeichnet, daß man Verbindungen gemäß Anspruch 1 disproportionieren läßt.

7. Verfahren gemäß Anspruch 6, dadurch gekennzeichnet, daß man die Tetrasulfide gemäß Formel I über ihren Schmelzpunkt hinaus erhitzt.

8. Verfahren gemäß Anspruch 6, dadurch gekennzeichnet, daß man die Tetrasulfide gemäß Formel I in einem inerten organischen Lösungsmittel löst und die Disproportionierungsreaktion im Temperaturbereich zwischen 20 °C und Siedepunkt des Lösungsmittels ablaufen läßt.

9. Verfahren zur Herstellung von Gemischen bestehend aus Verbindungen gemäß Anspruch 2, dadurch gekennzeichnet, daß man eine wässrige alkalische Lösung der entsprechenden N,N'-substituierten 2,4-Diamino-6-mercaptotriazine in einem Zweiphasensystem mit einer Lösung von $S_2Cl_2$ in einem inerten, die entstehenden Tetrasulfide lösenden organischen Lösungsmittel umsetzt.

10. Verfahren gemäß Anspruch 9, dadurch gekennzeichnet, daß man als Lösungsmittel chlorierte Kohlenwasserstoffe, Ether oder Ester sowie aromatische Kohlenwasserstoffe und Ketone verwendet, die wasserunlöslich sind.

11. Verwendung von Verbindungen oder deren Gemischen gemäß den Ansprüchen 1 - 10 als Beschleuniger in vulkanisierbaren, Füllstoffe, Schwefel und weitere übliche Bestandteile enthaltenden Mischungen auf der Basis eines oder mehrerer natürlicher und/oder synthetischer Kautschuk(e)(s), die gegebenenfalls weitere Verbindungen gemäß Anspruch 1 beziehungsweise konventionelle Beschleuniger, insbesondere Sulfenamide, Dibenzthiazolydisulfid und/oder Thiurame, Schwefelspender und/oder Verzögerer und/oder Organosilane enthalten.

12. Verwendung der Verbindungen oder deren Gemischen gemäß den Ansprüchen 1 - 10 als Vernetzer in vulkanisierbaren, schwefelfreien, Füllstoffe und weitere übliche Bestandteile enthaltenden Mischungen auf der Basis eines oder mehrerer natürlicher und/oder synthetischer Kautschuk(e)(s), die gegebenenfalls weitere Verbindungen gemäß Anspruch 1 beziehungsweise konventionelle Beschleuniger, insbesondere Sulfenamide, Dibenzthiazolyldisulfid und/oder Thiurame, Schwefelspender und/oder Verzögerer und/oder Organosilane enthalten.

13. Vulkanisierbare, Füllstoffe, Schwefel und weitere übliche Bestandteile enthaltende Mischungen auf der Basis eines oder mehrerer natürlicher und/oder synthetischer Kautschuk(e)(s), dadurch gekennzeichnet, daß sie 0,01 - 10 Teile, bevorzugt 0,1 - 5 Teile, pro 100 Teile Kautschuk, der Verbindungen gemäß den Ansprüchen 1 - 10 sowie Gemische derselben und/oder Gemische mit konventionellen Beschleunigern enthalten bei Schwefeldosierungen von 0,1 - 10 Teilen pro 100 Teile Kautschuk.

14. Vulkanisierbare Mischungen gemäß Anspruch 13, dadurch gekennzeichnet, daß sie den Verzögerer N-Phenyl-N-(trichlormethylsulfenyl)-benzolsulfonamid und die Verbindungen gemäß den Ansprüchen 1 - 10 bzw. deren Gemische im Molverhältnis 0,5 - 1,5 : 1, vorzugsweise 0,8 - 1,2 : 1, bei einer Schwefeldosierung von 0,1 - 10 Gewichtsteilen auf 100 Teile Kautschuk, vorzugsweise 0,5 - 8 Teile bezogen auf 100 Teile Kautschuk, enthalten.

15. Vulkanisierbare, Füllstoffe und weitere übliche Bestandteile enthaltende, schwefelfreie Mischungen auf der Basis eines oder mehrerer natürlicher und/oder synthetischer Kautschuke, dadurch gekennzeichnet, daß sie 0,2 - 15 Teile, bevorzugt0,3 - 8 Teile, der Verbindungen oder deren Gemische gemäß den Ansprüchen 1 - 10 auf 100 Teile Kautschuk enthalten oder Gemische mit konventionellen Beschleunigern.

16. Vulkanisierbare Mischungen gemäß Anspruch 15, dadurch gekennzeichnet, daß sie den Verzögerer N-Phenyl-N-(trichlormethylsulfenyl)-benzol-sulfonamid und die Verbindungen gemäß den Ansprüchen 1 - 10 beziehungsweise deren Gemische im Molverhältnis 0,5 - 1,5 : 1, vorzugsweise 0,8 - 1,2 : 1, auf 100 Teile Kautschuk enthalten.

17. Vulkanisierbare Mischungen gemäß den Ansprüchen 13 bis 16, dadurch gekennzeichnet, daß sie als Füllstoffe nur Kieselsäuren enthalten.

**18.** Vulkanisierbare Mischungen gemäß den Ansprüchen 13 bis 16, dadurch gekennzeichnet, daß sie als Füllstoff außer Ruß noch mehr als 15 Teile Kieselsäure pro 100 Teile Kautschuk enthalten.

## Claims

**1.** Compounds corresponding to the following general formula:

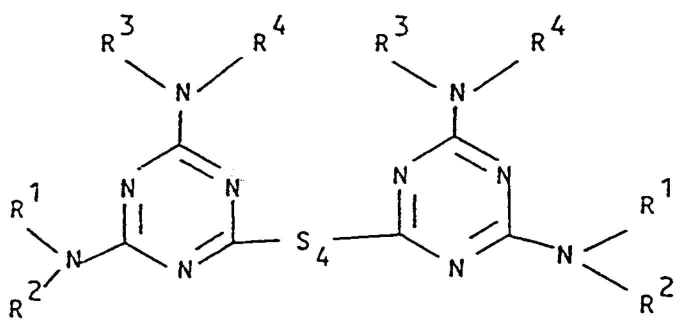

(I)

in which
$R^1$, $R^2$ = H, $R^2$ = benzyl,
$R^2$, $R^3$, $R^4$ = $C_{1-8}$ alkyl, allyl, $C_{3-8}$ cycloalkyl unsubstituted or substituted by 1 to 3 methyl groups, 2-hydroxyethyl, 3-hydroxypropyl, 2-hydroxypropyl,
except for bis-(2-ethylamino-4-diethylamino-3-triazin-6-yl)-tetrasulfide.

**2.** Mixtures consisting of compounds corresponding to the following general formula:

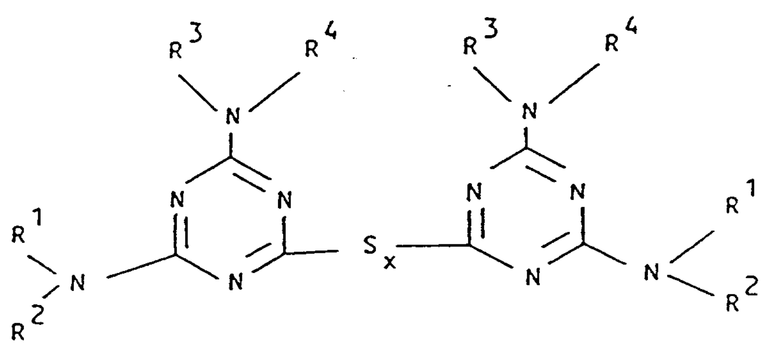

(II)

in which $R^1$, $R^2$, $R^3$ and $R^4$ have the same meanings as in claim 1 and $S_x$ corresponds to an average statistical chain length with x = 4,
except for bis-(2-ethylamino-4-diethylamino-s-triazin-6-yl)-tetrasulfide.

**3.** A process for the production of the tetrasulfides claimed in claim 1, characterized in that an aqueous alkaline solution of the corresponding N,N'-substituted 2,4-diamino-6-mercaptotriazines is reacted in a two-phase system with a solution of $S_2Cl_2$ in an inert organic solvent with little or no dissolving effect on the tetrasulfide formed at temperatures in the range from -5 °C to < + 20 °C, the ratio of mercaptotriazine to $S_2Cl_2$ being 2:1 to 2:1.2.

**4.** A process as claimed in claim 3, characterized in that alkali metal hydroxide is used in at least the stoichiometric quantity required for the reaction.

**5.** A process as claimed in claims 3 and 4, characterized in that sodium hydroxide or potassium hydroxide is used, $C_{5-10}$ alkanes or $C_{5-8}$ cycloalkanes, optionally substituted by 1 to 3 methyl groups, being used as the solvent.

6. A process for the production of the mixtures of compounds claimed in claim 2, characterized in that the compounds claimed in claim 1 are disproportionated.

7. A process as claimed in claim 6, characterized in that the tetrasulfides corresponding to formula I are heated beyond their melting point.

8. A process as claimed in claim 6, characterized in that the tetrasulfides corresponding to formula I are dissolved in an inert organic solvent and the disproportionation reaction is carried out at a temperature in the range from 20°C to the boiling point of the solvent.

9. A process for the production of the mixtures of compounds claimed in claim 2, characterized in that an aqueous alkaline solution of the corresponding N,N'-substituted 2,4-diamino-6-mercaptotriazines is reacted in a two-phase system with a solution of $S_2Cl_2$ in an inert organic solvent which dissolves the tetrasulfides formed.

10. A process as claimed in claim 9, characterized in that chlorinated hydrocarbons, ethers or esters and aromatic hydrocarbons and ketones insoluble in water are used as the solvent.

11. The use of the compounds according to claims 1 to 10 or mixtures thereof as accelerators in vulcanizable mixtures containing fillers, sulfur and other typical ingredients based on one or more natural and/or synthetic rubber(s) which optionally contain further compounds according to claim 1 or conventional accelerators, more particularly sulfenamides, dibenzthiazolyl disulfide and/or thiurams, sulfur donors and/or retarders and/or organosilanes.

12. The use of the compounds according to claims 1 to 10 or mixtures thereof as crosslinking agents in vulcanizable, sulfur-free mixtures containing fillers and other typical ingredients based on one or more natural and/or synthetic rubber(s) which optionally contain further compounds according to claim 1 or conventional accelerators, more particularly sulfenamides, dibenzthiazolyl disulfide and/or thiurams, sulfur donors and/or retarders and/or organosilanes.

13. Vulcanizable mixtures containing fillers, sulfur and other typical ingredients based on one or more natural and/or synthetic rubber(s), characterized in that they contain 0.01 to 10 parts and preferably 0.1 to 5 parts per 100 parts rubber of the compounds according to claims 1 to 10 and mixtures thereof and/or mixtures with conventional accelerators for sulfur dosages of 0.1 to 10 parts per 100 parts rubber.

14. Vulcanizable mixtures as claimed in claim 13, characterized in that they contain the retarder N-phenyl-N-(trichloromethylsulfenyl)-benzenesulfonamide and the compounds according to claims 1 to 10 or mixtures thereof in a molar ratio of 0.5-1.5:1 and preferably in a molar ratio of 0.8-1.2:1 for a sulfur dosage of 0.1 to 10 parts by weight to 100 parts rubber and preferably 0.5 to 8 parts to 100 parts rubber.

15. Vulcanizable sulfur-free mixtures containing fillers and other typical ingredients based on one or more natural and/or synthetic rubbers, characterized in that they contain 0.2 to 15 parts and preferably 0.3 to 8 parts of the compounds according to claims 1 to 10 or mixtures thereof to 100 parts rubber or mixtures with conventional accelerators.

16. Vulcanizable mixtures as claimed in claim 15, characterized in that they contain the retarder N-phenyl-N-(trichloromethylsulfenyl)-benzenesulfonamide and the compounds according to claims 1 to 10 or mixtures thereof in a molar ratio of 0.5-1.5:1 and preferably 0.8-1.2:1 to 100 parts rubber.

17. Vulcanizable mixtures as claimed in claims 13 to 16, characterized in that they only contain silicas as fillers.

18. Vulcanizable mixtures as claimed in claims 13 to 16, characterized in that, in addition to carbon black, they contain more than 15 parts silica to 100 parts rubber as filler.

## Revendications

**1.** de formule générale (I) :

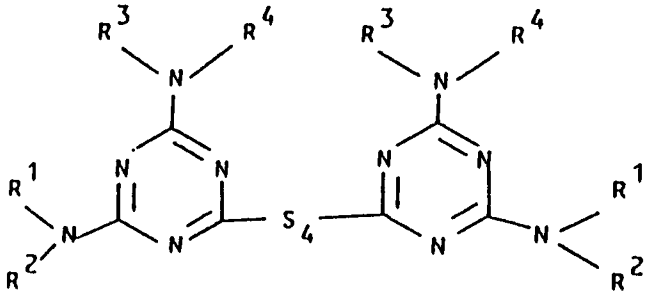

(I)

dans laquelle :
$R^1$, $R^2$ = H, $R^2$ = benzyle,
$R^2$, $R^3$, $R^4$ = alkyle $C_1$-$C_8$, allyle, cycloalkyle $C_3$-$C_8$, ce dernier étant non substitué ou substitué par 1-3 groupes méthyle, 2-hydroxyéthyle, 3-hydroxy-propyle, 2-hydroxypropyle,
à l'exception du bis-(2-éthylamino-4-diéthylamino-s-triazin-6-yl)tétrasulfure.

**2.** Mélanges constitués de composés de formule générale (II) :

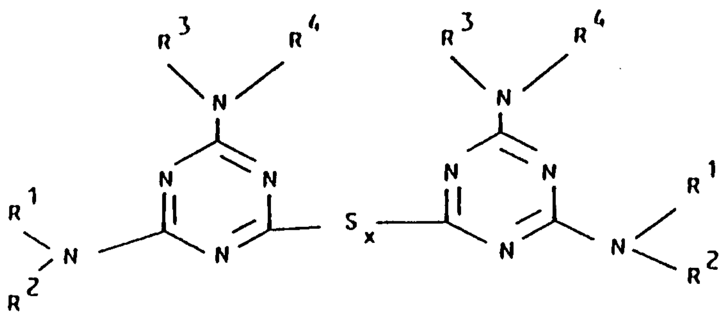

(II)

dans laquelle $R^1$, $R^2$, $R^3$ et $R^4$ ont les mêmes significations qu'à la revendication 1 et $S_x$ a une longueur moyenne statistique de chaîne correspondant à x = 4, à l'exception du bis-(2-éthylamino-4-diéthylamino-s-triazin-6-yl)tétrasulfure.

**3.** Procédé de préparation des tétrasulfures selon la revendication 1, caractérisé en ce qu'on fait réagir une solution aqueuse alcaline des 2,4-diamino-6-mercaptotriazines N-N'- substituées correspondantes dans un système biphasé avec une solution de $S_2Cl_2$ dans un solvant organique inerte ne dissolvant pas ou peu le tétrasulfure formé, à des températures comprises entre -5 °C et < + 20 °C, la proportion de mercaptotriazine par rapport à $S_2Cl_2$ étant comprise entre 2:1 et 2:1,2.

**4.** Procédé de préparation des tétrasulfures selon la revendication 3, caractérisé en ce qu'on utilise au moins la quantité stoechiométrique d'hydroxyde alcalin nécessaire à la réaction.

**5.** Procédé selon les revendications 3 à 4, caractérisé en ce qu'on utilise de l'hydroxyde de sodium ou de potassium et comme solvant des alcanes $C_5$-$C_{10}$ ou cycloalcanes $C_5$-$C_8$, le cas échéant substitués par 1-3 groupes méthyle.

**6.** Procédé de préparation de mélanges constitués de composés selon la revendication 2, caractérisé en ce qu'on dismute des composés selon la revendication 1.

**7.** Procédé selon la revendication 6, caractérisé en ce qu'on chauffe au-delà de leur point de fusion les tétrasulfures selon la formule (I).

8. Procédé selon la revendication 6, caractérisé en ce qu'on dissout les tétrasulfures selon la formule (I) dans un solvant organique inerte et qu'on réalise la réaction de dismutation dans l'intervalle de température compris entre 20 °C et le point d'ébullition du solvant.

9. Procédé de préparation de mélanges constitués de composés selon la revendication 2, caractérisé en ce qu'on fait réagir une solution alcaline aqueuse des 2,4-diamino-6-mercaptotriazine N, N'- substituées correspondantes dans un système biphasé avec une solution de $S_2Cl_2$ dans un solvant inerte organique dissolvant les tétrasulfures formés.

10. Procédé selon la revendication 9, caractérisé en ce qu'on utilise comme solvants des hydrocarbures chlorés, des éthers ou esters ainsi que des hydrocarbures aromatiques et des cétones qui sont insolubles dans l'eau.

11. Utilisation des composés ou de leurs mélanges selon les revendications 1-10 comme accélérateurs dans les mélanges vulcanisables contenant des charges, du soufre et d'autres composants usuels à base d'un ou de plusieurs caoutchoucs naturels et/ou synthétiques qui contiennent le cas échéant d'autres composés selon la revendication 1, ou des accélérateurs classiques, notamment des sulfénamides, dibenzothiazolyldisulfure et/ou thiurames, des donneurs de soufre et/ou des retardateurs et/ou des organosilanes.

12. Utilisation des composés ou de leurs mélanges selon les revendications 1-10, comme agents réticulants dans les mélanges vulcanisables, sans soufre contenant des charges et d'autres composants usuels à base d'un ou de plusieurs caoutchoucs naturels ou synthétiques qui contiennent le cas échéant d'autres composés selon la revendication 1 ou des accélérateurs classiques notamment des sulfamides, dibenzothiazolyldisulfure, et/ou thiurames, des donneurs de soufre et/ou retardateurs et/ou des organosilanes.

13. Mélanges vulcanisables contenant des charges, du soufre et d'autres composants usuels à base d'un ou de plusieurs caoutchoucs naturels et/ou synthétiques,
caractérisés en ce qu'ils contiennent 0,01-10 parties, de préférence 0,1-5 parties, pour 100 parties de caoutchouc, des composés selon les revendications 1-10 ainsi que leurs mélanges et/ou des mélanges avec des accélérateurs classiques, pour des teneurs en soufre de 0,1-10 parties pour 100 parties de caoutchouc.

14. Mélanges vulcanisables selon la revendication 13, caractérisés en ce qu'ils contiennent le retardant N-phényl-N-(trichlorométhylsulfényl)-benzène-sulfonamide et les composés selon les revendications 1-10 ou leurs mélanges en proportion molaire 0,5-1,5:1 de préférence 0,8-1,2:1 pour une teneur en soufre de 0,1-10 parties en poids pour 100 parties de caoutchouc, de préférence 0,5-8 parties rapportées à 100 parties de caoutchouc.

15. Mélanges sans soufre vulcanisables, contenant des charges et d'autres composants usuels à base d'un ou plusieurs caoutchoucs naturels et/ou synthétiques caractérisés en ce qu'ils contiennent 0,2-15 parties, de préférence 0,3-8 parties des composés ou de leurs mélanges selon les revendications 1-10 pour 100 parties de caoutchouc ou des mélanges avec des accélérateurs classiques.

16. Mélanges vulcanisables selon la revendication 15, caractérisés en ce qu'ils contiennent le retardateur N-phényl-N-(trichlorométhylsulfényl)-benzène-sulfonamide et les composés selon les revendications 1-10 ou leurs mélanges en proportion molaire 0,5-1,5:1, de préférence 0,8-1,2 : 1 pour 100 parties de caoutchouc.

17. Mélanges vulcanisables selon les revendications 13 à 16, caractérisés en ce qu'ils ne contiennent comme charge que des acides siliciques.

18. Mélanges vulcanisables selon les revendications 13 à 16, caractérisés en ce qu'ils contiennent comme charge outre le noir de carbone plus de 15 parties d'acide silicique pour 100 parties de caoutchouc.